# DEMANDE DE BREVET EUROPEEN

(11) **EP 1 757 579 A1**
(43) Date de publication de la demande: **28.02.2007**
(21) Numéro de dépôt: 06291332.2
(22) Date de dépôt: 21.08.2006
(51) Int. Cl.: C07C 211/45, C07C 211/51, C07C 217/76, C07C 217/84, C07C 237/30, C07C 245/08, C07C 251/68, C07C 309/46, C07D 233/54, C07D 295/12, A61K 8/41, A61Q 5/10

(54) **Para-phénylènediamines primaires 2,6-disubstituées et leur utilisation en teinture d'oxydation de fibres kératiniques**

(30) Priorité: 22.08.2005 FR 0508666
(71) Demandeur: L'Oreal, 75008 Paris (FR)
(72) Inventeur: Ramos-Stanbury, Laure, 92330 Sceaux (FR); Sabelle, Stéphane, 75005 Paris (FR); Ly-Carry, Thy-My, 94100 Saint-Maur-Des-Fosses (FR)
(74) Mandataire: Casalonga, Axel

(57) **Abrégé**

L'invention concerne des composés para-phénylènediamine 2,6-disubstituées de formule (I), leurs sels et solvates : 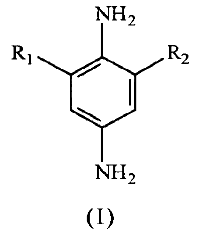 dans laquelle R₁ et R₂ représentent, indépendamment,
-un radical alkyle linéaire ou ramifié en C₁-C₆ éventuellement substitué par un radical alcoxy en C₁-C₆, hydroxy, amino, alkyle(C₁-C₆)amino, dialkyl(C₁-C₆)amino ou encore par un radical cyclique de type : 1-pyrrolidinyle, 1-cyclohexylaminyle, 1-pipérazinyle, 1-diazépanyle, 1-morpholinyle, 1-imidazoyle, ce cycle pouvant être substitué par un ou plusieurs groupements alkyle, hydroxy ou amino ;
-un radical alcoxy, linéaire ou ramifié, en C₁-C₆, monohydroxyalcoxy en C₁-C₆, polyhydroxyalcoxy en C₁-C₆;
-un radical carboxylique
-un radical amide.

L'invention porte également sur des composés intermédiaires, sur une composition pour la teinture d'oxydation des fibres kératiniques, et en particulier des fibres kératiniques humaines telles que les cheveux, et sur des procédés de teinture d'oxydation mettant en oeuvre ladite composition.

## Description

La présente invention est relative à de nouvelles para-phénylènediamines primaires 2,6-disubstituées, à une composition pour la teinture d'oxydation des fibres kératiniques, et en particulier des fibres kératiniques humaines telles que les cheveux, comprenant au moins, comme base d'oxydation, une para-phénylènediamine primaire 2,6-disubstituée, et aux procédés de teinture d'oxydation la mettant en oeuvre.

Il est connu de teindre les fibres kératiniques et en particulier les cheveux humains avec des compositions tinctoriales contenant des précurseurs de colorant d'oxydation, en particulier des ortho- ou para-phénylènediamines, des ortho- ou para-aminophénols, des composés hétérocycliques tels que des dérivés de diaminopyrazole, appelés généralement bases d'oxydation. Les précurseurs de colorants d'oxydation, ou bases d'oxydation, sont des composés incolores ou faiblement colorés qui, associés à des produits oxydants, peuvent donner naissance par un processus de condensation oxydative à des composés colorés et colorants.

On sait également que l'on peut faire varier les nuances obtenues avec ces bases d'oxydation en les associant à des coupleurs ou modificateurs de coloration, ces derniers étant choisis notamment parmi les méta-diamines aromatiques, les méta-aminophénols, les méta-diphénols et certains composés hétérocycliques.

La variété des molécules mises en jeu au niveau des bases d'oxydation et des coupleurs, permet l'obtention d'une riche palette de couleurs.

La coloration dite "permanente" obtenue grâce à ces colorants d'oxydation, doit par ailleurs satisfaire à un certain nombre d'exigences. Ainsi, elle doit être sans inconvénient sur le plan toxicologique, elle doit permettre d'obtenir des nuances dans l'intensité souhaitée, présenter une bonne tenue aux agents extérieurs (lumière, intempéries, lavage, ondulation permanente, transpiration, frottements).

Les colorants doivent également permettre de couvrir les cheveux blancs, et être enfin les moins sélectifs possibles, c'est-à-dire permettre d'obtenir des écarts de coloration les plus faibles possibles tout au long d'une même fibre kératinique, qui peut être en effet différemment sensibilisée (c'est-à-dire abîmée) entre sa pointe et sa racine. Ils doivent également présenter une bonne stabilité chimique dans les formulations. Ils doivent présenter un bon profil toxicologique.

De plus, pour un certain nombre d'applications, on recherche des colorants donnant sur les cheveux des nuances chromatiques.

Or, la demanderesse vient maintenant de découvrir, de façon avantageuse et surprenante, qu'il était possible d'obtenir des teintures, capables de conduire à des colorations puissantes, peu sélectives, et présentant d'excellentes propriétés de résistance aux diverses agressions que peuvent subir les fibres kératiniques en utilisant à titre de base d'oxydation les para-phénylènediamines primaires 2,6-disubstituées de formule (I) ci-après ou leurs sels ou solvates physiologiquement acceptables.

La présente invention a donc pour premier objet les nouvelles para-phénylènediamines primaires 2,6-disubstituées de formule (I) suivante : 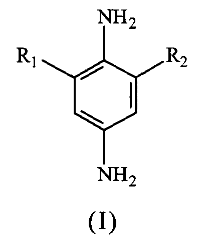 dans laquelle R₁ et R₂ représentent, indépendamment,
- un radical alkyle linéaire ou ramifié en C₁-C₆ éventuellement substitué par un radical alcoxy en C₁-C₆, hydroxy, amino, alkyle(C₁-C₆)amino, dialkyl(C₁-C₆)amino ou encore par un radical cyclique de type :1-pyrrolidinyle, 1-cyclohexylaminyle, 1-pipérazinyle, 1-diazépanyle, 1-morpholinyle, 1-imidazoyle, ce cycle pouvant être substitué par un ou plusieurs groupements alkyle, hydroxy ou amino ;
- un radical alcoxy, linéaire ou ramifié, en C₁-C₆, monohydroxyalcoxy en C₁-C₆, polyhydroxyalcoxy en C₁-C₆;
- un radical carboxylique
- un radical amide.
à l'exception des composés suivants :

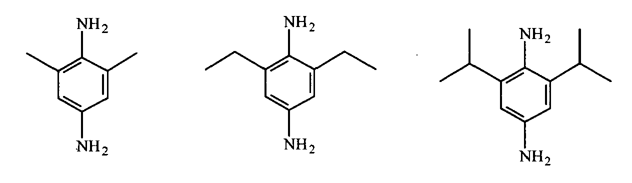

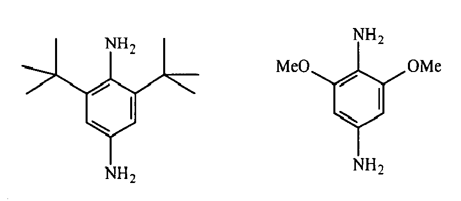

L'invention a également pour objet les solvates et sels d'acides organiques ou minéraux physiologiquement acceptables des composés de formule (I).

D'une manière générale, les sels d'addition utilisables sont notamment choisis parmi les sels d'addition avec un acide, tels que l'acide chlorhydrique, l'acide bromhydrique, l'acide sulfurique, l'acide citrique, l'acide succinique, l'acide tartrique, l'acide lactique, l'acide méthanesulfonique, l'acide para-toluènesulfonique, l'acide benzènesulfonique, l'acide phosphorique et l'acide acétique.

Ils peuvent aussi être sous forme de solvates par exemple un hydrate ou un solvate d'alcool linéaire ou ramifié tel que l'éthanol ou l'isopropanol.

Selon un premier mode préféré, R₁ et R₂ représentent indépendamment :
- un radical méthyle, éthyle, n-propyle, iso-propyle, sec-butyle, iso-butyle, ter-butyle, (2,2'diméthyl)butyle, imidazopropyle, diméthylaminopropyle ;
- un radical méthoxybutyle, aminométhyle, (3-diméthylamino)propyle, imidazolo-n-propyle, pyrrolidinoéthyle ;
- un radical méthoxy, hydroxyméthyle, α-hydroxyéthyle, β-hydroxyéthyle, β-hydroxy-iso-propyle ;
- un radical carboxylique ;
- un radical amido.

A titre de para-phénylènediamines de formule (I), on peut citer les composés suivants ainsi que leurs sels et/ou solvates.

| | |
|---|---|
| 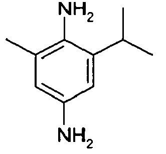 | 2-Isopropyl-6-méthyl-benzène-1,4-diamine |
| 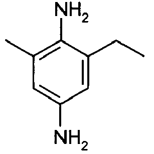 | 2-Ethyl-6-méthyl-benzène-1,4-diamine |
| 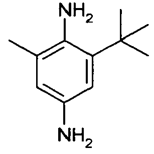 | 2-tert-Butyl-6-méthyl-benzène-1,4-diamine |
| 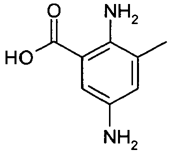 | Acide 2,5-diamino-3-méthyl-benzoïque |
| 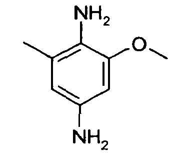 | 2-Méthoxy-6-méthyl-benzène-1,4-diamine |
| 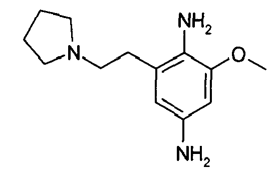 | 2-Méthoxy-6-(2-pyrrolidin-1-yl-ethyl)-benzène-1,4-diamine |
| 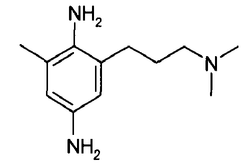 | 2-(3-Diméthylamino-propyl)-6-méthyl -benzène-1,4-diamine |
| 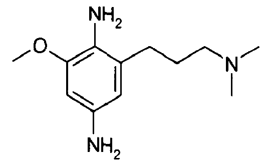 | 2-(3-Diméthylamino-propyl)-6-méthoxy-benzène-1,4-diamine |
| 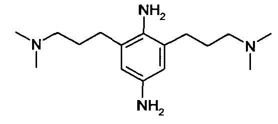 | 2,6-Bis-(3-diméthylamino-propyl)-benzène-1,4-diamine |
| 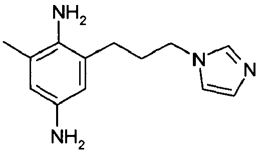 | 2-(3-Imidazol-1-yl-propyl)-6-methyl -benzene-1,4-diamine |
| 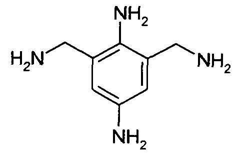 | 2,6-Bis-aminométhyl-benzène-1,4-diamine |
| 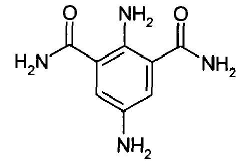 | 2,5-Diamino-isophthalamide |
| 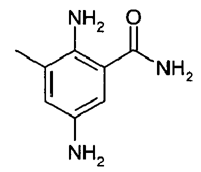 | 2,5-Diamino-3-méthylbenzamide |
| 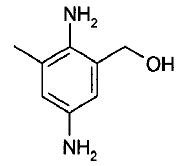 | (2,5-Diamino-3-méthyl-phényl)-méthanol |
| 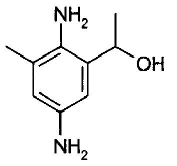 | 1-(2,5-Diamino-3-méthyl-phényl)-éthanol |
| 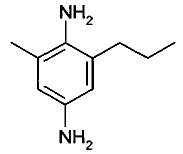 | 2-Méthyl-6-propyl-benzène-1,4-diamine |
| 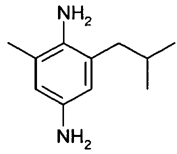 | 2-Isobutyl-6-méthyl-benzène-1,4-diamine |
| 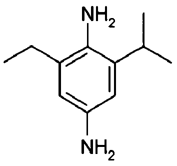 | 2-Ethyl-6-isopropyl-benzène-1,4-diamine |
| 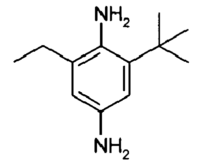 | 2-tert-Butyl-6-éthyl-benzène-1,4-diamine |
| 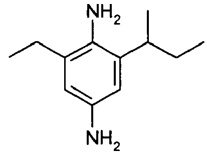 | 2-sec-Butyl-6-éthyl-benzène-1,4-diamine |
| 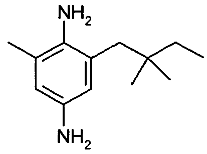 | 2-(2,2-Diméthyl-butyl)-6-méthyl-ben zene-1,4-diamine |
| 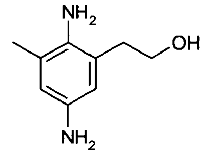 | 2-(2,5-Diamino-3-méthyl-phényl)-éthanol |
| 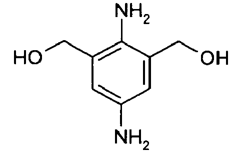 | (2,5-Diamino-3-hydroxyméthyl-phényl)-méthanol |
| 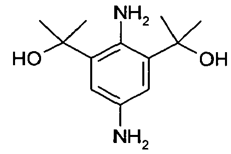 | 2-[2,5-Diamino-3-(1-hydroxy-1-méthy 1-éthyl)-phényl]-propan-2-ol |
| 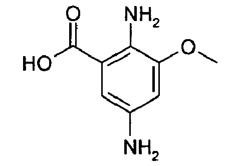 | Acide 2,5-diamino-3-méthoxy-benzoïque |
| 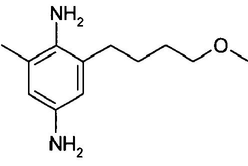 | 2-(4-Méthoxy-butyl)-6-méthyl-benzène-1,4-diamine |

Les para-phénylènediamines primaires 2,6-substituées de formule (I) plus particulièrement préférées sont choisies parmi la 2-isopropyl-6-méthyl-benzène-1,4-diamine, la 2-éthyl-6-méthyl-benzène-1,4-diamine, la 2-tert-butyl-6-méthyl-benzène-1,4-diamine, l'Acide 2,5-diamino-3-méthyl-benzoïque, la 2-méthoxy-6-méthyl-benzène-1,4-diamine, la 2-méthoxy-6-(2-pyrrolidin-1-yl-éthyl)-benzène-1,4-diamine, la 2-(3-diméthylamino-propyl)-6-méthyl-benzène-1,4-diamine, la 2-(3-imidazol-1-yl-propyl)-6-méthyl-benzène-1,4-diamine, le 2,5-diamino-isophthalamide, le 2,5-diamino-3-méthylbenzamide, le 1-(2,5-diamino-3-méthyl-phényl)-éthanol, la 2-méthyl-6-propyl-benzène-1,4-diamine, la 2-isobutyl-6-méthyl-benzène-1,4-diamine, le 2-(2,5-Diamino-3-méthyl-phényl)-éthanol ou l'un de leurs sels et/ou solvates physiologiquement acceptables.

La présente demande vise également un procédé de synthèse d'un composé para-phénylènediamine de formule (I) tel que définit précédemment qui comprend une étape de réduction du composé intermédiaire de formule (II) correspondant ou du composé intermédiaire de formule (III) correspondant ou du composé intermédiaire de formule (IV) correspondant: 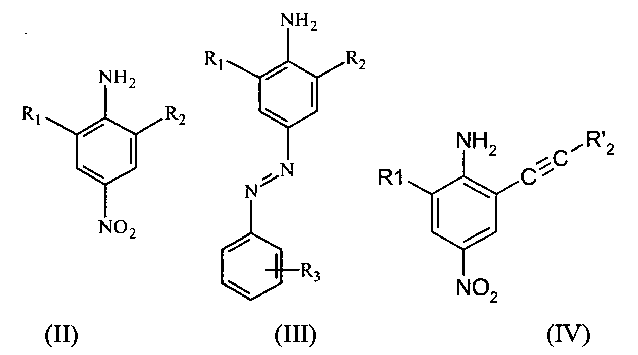 dans laquelle R₁ et R₂ représentent, indépendamment,
- un radical alkyle linéaire ou ramifié en C₁-C₆ éventuellement substitué par un radical alcoxy en C₁-C₆, hydroxy, amino, alkyle(C₁-C₆)amino, dialkyl(C₁-C₆)amino ou encore par un radical cyclique de type :1-pyrrolidinyle, 1-cyclohexylaminyle, 1-pipérazinyle, 1-diazépanyle, 1-morpholinyle, 1-imidazoyle, ce cycle pouvant être substitué par un ou plusieurs groupements alkyle, hydroxy ou amino ;
- un radical alcoxy, linéaire ou ramifié, en C₁-C₆, monohydroxyalcoxy en C₁-C₆, polyhydroxyalcoxy en C₁-C₆;
- un radical carboxylique
- un radical amide ;
R'₂ représentant
- un radical alkyle linéaire ou ramifié en C₁-C₄ éventuellement substitué par un radical alcoxy en C₁-C₆, hydroxy, amino, alkyle(C₁-C₆)amino, dialkyl(C₁-C₆)amino ou encore par un radical cyclique de type :1-pyrrolidinyle, 1-cyclohexylaminyle, 1-pipérazinyle, 1-diazépanyle, 1-morpholinyle, 1-imidazoyle, ce cycle pouvant être substitué par un ou plusieurs groupements alkyle, hydroxy ou amino ;
- un radical alcoxy, linéaire ou ramifié, en C₁-C₆, monohydroxyalcoxy en C₁-C₆, polyhydroxyalcoxy en C₁-C₆;
R₃ étant un hydrogène ou un groupe sulfonique.

La présente demande vise aussi les composés intermédiaires de formule (II) : 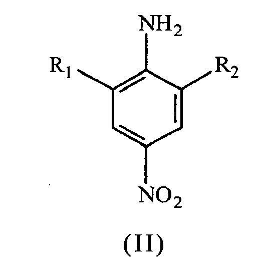 dans laquelle R₁ et R₂ représentent, indépendamment,
- un radical alkyle linéaire ou ramifié en C₁-C₆ éventuellement substitué par un radical alcoxy en C₁-C₆, hydroxy, amino, alkyle(C₁-C₆)amino, dialkyl(C₁-C₆)amino ou encore par un radical cyclique de type :1-pyrrolidinyle, 1-cyclohexylaminyle, 1-pipérazinyle, 1-diazépanyle, 1-morpholinyle, 1-imidazoyle, ce cycle pouvant être substitué par un ou plusieurs groupements alkyle, hydroxy ou amino ;
- un radical alcoxy, linéaire ou ramifié, en C₁-C₆, monohydroxyalcoxy en C₁-C₆, polyhydroxyalcoxy en C₁-C₆;
- un radical carboxylique
- un radical amide.
à l'exception des composés suivants :

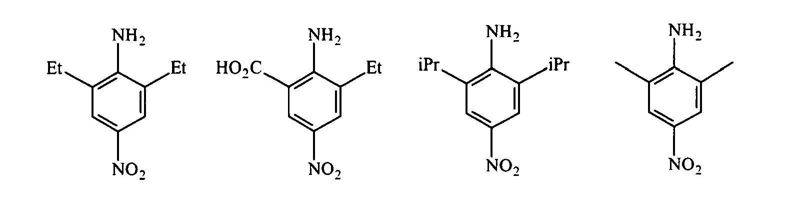

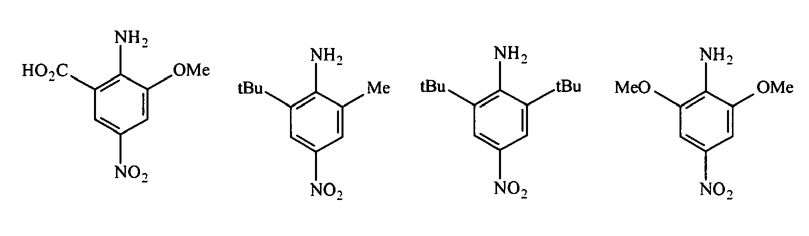

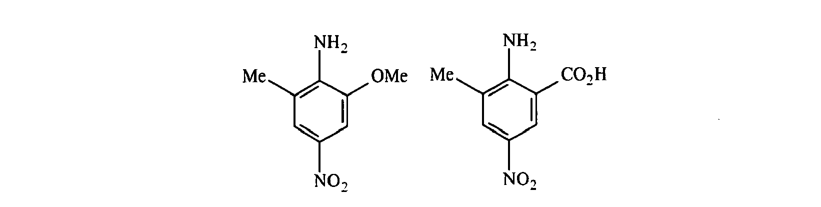

La présente demande vise aussi les composés intermédiaires de formule (III) : 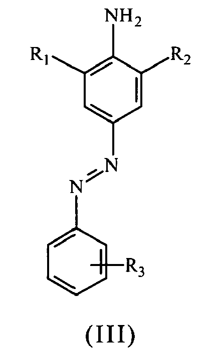 dans laquelle R₁ et R₂ représentent, indépendamment,
- un radical alkyle linéaire ou ramifié en C₁-C₆ éventuellement substitué par un radical alcoxy en C₁-C₆, hydroxy, amino, alkyle(C₁-C₆)amino, dialkyl(C₁-C₆)amino ou encore par un radical cyclique de type :1-pyrrolidinyle, 1-cyclohexylaminyle, 1-pipérazinyle, 1-diazépanyle, 1-morpholinyle, 1-imidazoyle, ce cycle pouvant être substitué par un ou plusieurs groupements alkyle, hydroxy ou amino ;
- un radical alcoxy, linéaire ou ramifié, en C₁-C₆, monohydroxyalcoxy en C₁-C₆, polyhydroxyalcoxy en C₁-C₆;
- un radical carboxylique
- un radical amide.
R₃ étant un hydrogène ou un groupe sulfonique ;
à l'exception des composés suivants :

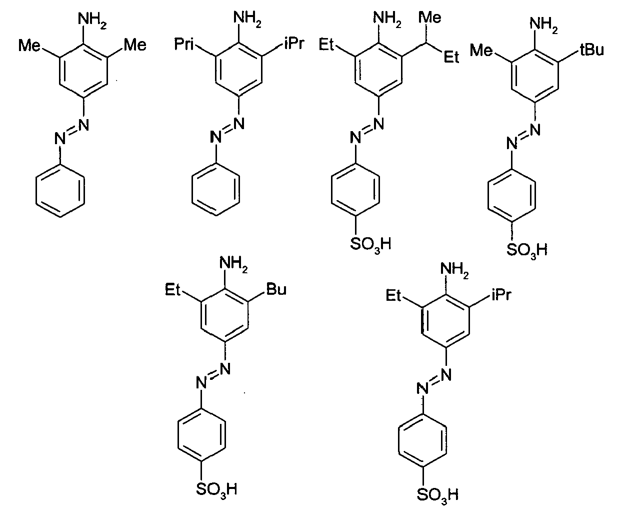

La présente demande vise aussi les composés intermédiaires de formule (IV) : 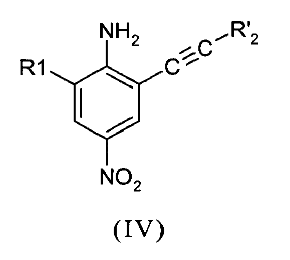 dans laquelle R₁ représente
- un radical alkyle linéaire ou ramifié en C₁-C₆ éventuellement substitué par un radical alcoxy en C₁-C₆, hydroxy, amino, alkyle(C₁-C₆)amino, dialkyl(C₁-C₆)amino ou encore par un radical cyclique de type :1-pyrrolidinyle, 1-cyclohexylaminyle, 1-pipérazinyle, 1-diazépanyle, 1-morpholinyle, 1-imidazoyle, ce cycle pouvant être substitué par un ou plusieurs groupements alkyle, hydroxy ou amino ;
- un radical alcoxy, linéaire ou ramifié, en C₁-C₆, monohydroxyalcoxy en C₁-C₆, polyhydroxyalcoxy en C₁-C₆;
- un radical carboxylique
- un radical amide ;
et R'₂ représente indépendamment :
- un radical alkyle linéaire ou ramifié en C₁-C₄ éventuellement substitué par un radical alcoxy en C₁-C₆ , hydroxy, amino, alkyle(C₁-C₆ )amino, dialkyl(C₁-C₆ )amino ou encore par un radical cyclique de type :1-pyrrolidinyle, 1-cyclohexylaminyle, 1-pipérazinyle, 1-diazépanyle, 1-morpholinyle, 1-imidazoyle, ce cycle pouvant être substitué par un ou plusieurs groupements alkyle, hydroxy ou amino ;
- un radical alcoxy, linéaire ou ramifié, en C₁-C₆ , monohydroxyalcoxy en C₁-C₆ , polyhydroxyalcoxy en C₁-C₆ ;
à l'exception du composé suivants:

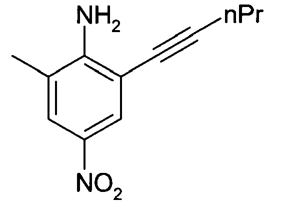

L'invention a également pour objet une composition pour la teinture d'oxydation des fibres kératiniques, et en particulier des fibres kératiniques humaines telles que les cheveux, caractérisée par le fait qu'elle contient, dans un milieu approprié pour la teinture, au moins un composé para-phénylènediamine de formule (I), ses sels et solvates: 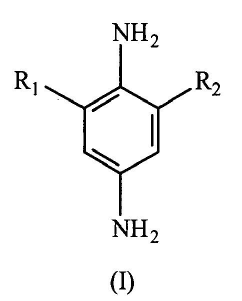 dans laquelle R₁ et R₂ représentent, indépendamment,
- un radical alkyle linéaire ou ramifié en C₁-C₆ éventuellement substitué par un radical alcoxy en C₁-C₆, hydroxy, amino, alkyle(C₁-C₆)amino, dialkyl(C₁-C₆)amino ou encore un radical cyclique de type :1-pyrrolidinyle, 1-cyclohexylaminyle, 1-pipérazinyle, 1-diazépanyle, 1-morpholinyle, 1-imidazoyle, ce cycle pouvant être substitué par un ou plusieurs groupements alkyle, hydroxy ou amino ;
- un radical alcoxy, linéaire ou ramifié, en C₁-C₆, monohydroxyalcoxy en C₁-C₆, polyhydroxyalcoxy en C₁-C₆;
- un radical carboxylique
- un radical amide.
à l'exception des composés suivants :

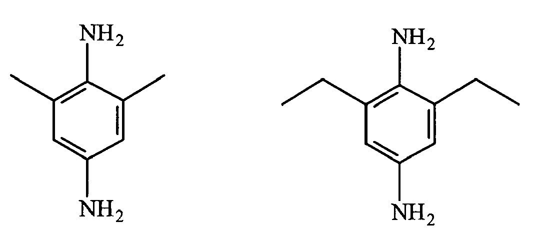

Les colorations obtenues avec la composition de teinture d'oxydation conforme à l'invention sont puissantes. Elles présentent, de plus, d'excellentes propriétés de résistance vis-à-vis de l'action des différents agents extérieurs tels que la lumière, les intempéries, le lavage, les ondulations permanentes, la transpiration, les frottements.

Un cinquième objet de la présente demande vise un procédé de teinture d'oxydation des fibres kératiniques mettant en oeuvre une telle composition tinctoriale.

D'autres caractéristiques, aspects, objets et avantages de la présente invention apparaîtront encore plus clairement à la lecture de la description et des exemples qui suivent.

A titre d'exemples de para-phénylènediamines primaires 2,6-substituées de formule (I) utilisables dans les compositions pour la teinture d'oxydation selon l'invention, on peut citer les composés suivants ainsi que leurs sels et/ou solvates :

| | |
|---|---|
| 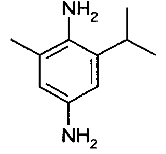 | 2-Isopropyl-6-méthyl-benzène-1,4-diamine |
| 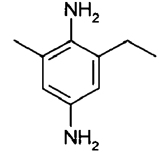 | 2-Ethyl-6-méthyl-benzène-1,4-diamine |
| 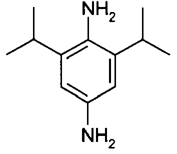 | 2,6-Diisopropyl-benzène-1,4-diamine |
| 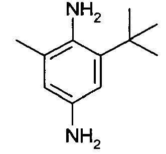 | 2-tert-Butyl-6-méthyl-benzène-1,4-diamine |
| 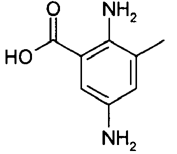 | Acide 2,5-diamino-3-méthyl-benzoïque |
| 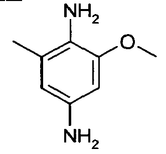 | 2-Méthoxy-6-méthyl-benzène-1,4-diamine |
| 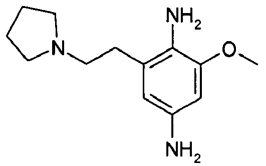 | 2-Méthoxy-6-(2-pyrrolidin-1-yl-ethyl)-benzène-1,4-diamine |
| 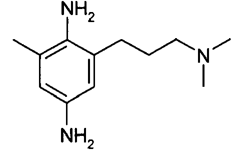 | 2-(3-Diméthylamino-propyl)-6-méthyl -benzène-1,4-diamine |
| 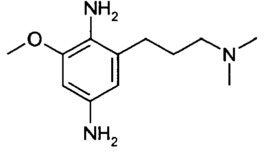 | 2-(3-Diméthylamino-propyl)-6-méthoxy-benzène-1,4-diamine |
| 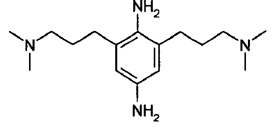 | 2,6-Bis-(3-diméthylamino-propyl)-benzène-1,4-diamine |
| 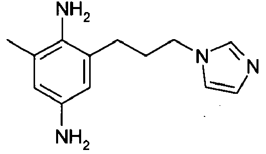 | 2-(3-Imidazol-1-yl-propyl)-6-methyl -benzene-1,4-diamine |
| 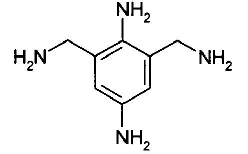 | 2,6-Bis-aminométhyl-benzène-1,4-diamine |
| 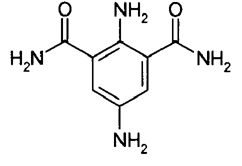 | 2,5-Diamino-isophthalamide |
| 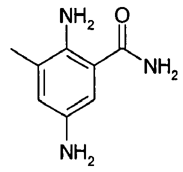 | 2,5-Diamino-3-méthylbenzamide |
| 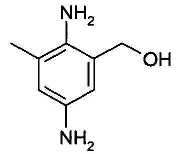 | (2,5-Diamino-3-méthyl-phényl)-méthanol |
| 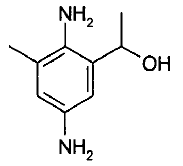 | 1-(2,5-Diamino-3-méthyl-phényl)-éthanol |
| 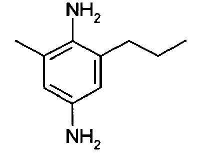 | 2-Méthyl-6-propyl-benzène-1,4-diamine |
| 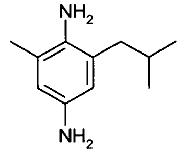 | 2-Isobutyl-6-méthyl-benzène-1,4-diamine |
| 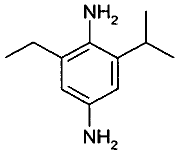 | 2-Ethyl-6-isopropyl-benzène-1,4-diamine |
| 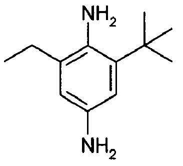 | 2-tert-Butyl-6-éthyl-benzène-1,4-diamine |
| 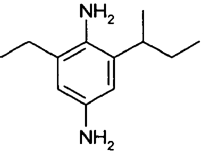 | 2-sec-Butyl-6-éthyl-benzène-1,4-diamine |
| 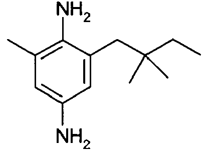 | 2-(2,2-Diméthyl-butyl)-6-méthyl-ben zene-1,4-diamine |
| 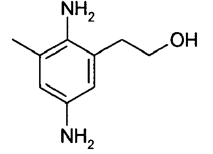 | 2-(2,5-Diamino-3-méthyl-phényl)-éthanol |
| 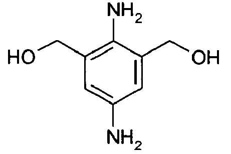 | (2,5-Diamino-3-hydroxyméthyl-phényl)-méthanol |
| 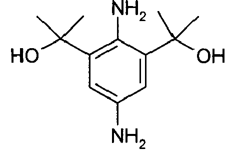 | 2-[2,5-Diamino-3-(1-hydroxy-1-méthy 1-éthyl)-phényl]-propan-2-ol |
| 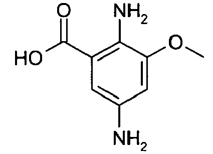 | Acide 2,5-diamino-3-méthoxy-benzoïque |
| 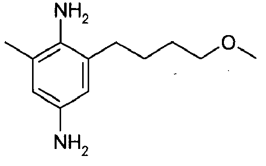 | 2-(4-Méthoxy-butyl)-6-méthyl-benzène-1,4-diamine |

Les para-phénylènediamines primaires 2,6-substituées de formule (I) plus particulièrement préférées utilisables dans les compositions pour la teinture d'oxydation sont :
la 2-isopropyl-6-méthyl-benzène-1,4-diamine, la 2-éthyl-6-méthyl-benzène-1,4-diamine, la 2-tert-butyl-6-méthyl-benzène-1,4-diamine, l'acide 2,5-diamino-3-méthyl-benzoïque, la 2-méthoxy-6-méthyl-benzène-1,4-diamine, la 2-méthoxy-6-(2-pyrrolidin-1-yl-éthyl)-benzène-1,4-diamine, la 2-(3-diméthylamino-propyl)-6-méthyl-benzène-1,4-diamine, la 2-(3-imidazol-1-yl-propyl)-6-méthyl-benzène-1,4-diamine, le 2,5-diamino-isophthalamide, le 2,5-diamino-3-méthylbenzamide, le 1-(2,5-diamino-3-méthyl-phényl)-éthanol, la 2-méthyl-6-propyl-benzène-1,4-diamine, la 2-isobutyl-6-méthyl-benzène-1,4-diamine, le 2-(2,5-Diamino-3-méthyl-phényl)-éthanol ou l'un de leurs sels ou solvates physiologiquement acceptables.

Les para-phénylènediamines primaires 2,6-substituées de formule (I) selon l'invention peuvent être préparées par exemple selon les trois voies de synthèse ci-dessous :
Voie de synthèse 1 : 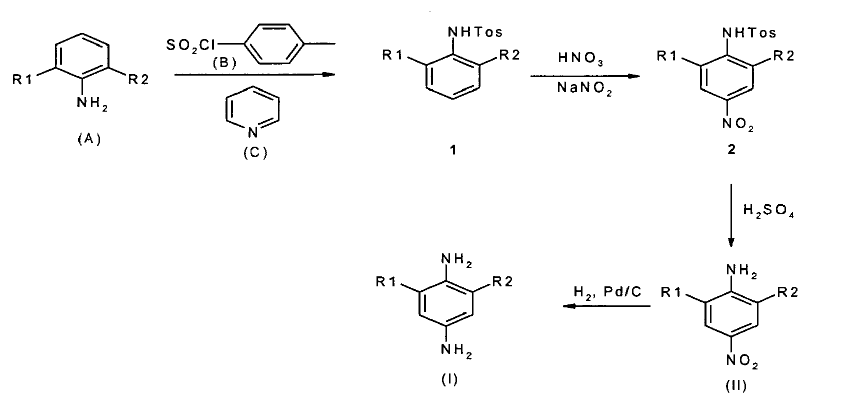 Les étapes de protection de l'amine par un groupe tosyle, de nitration et de déprotection de l'amine ont été réalisées selon la méthode décrite dans 1a publication Rec. Trav. Chim., 1954, 73, 809-818. La dernière étape permettant d'accéder aux composés (I) est une étape de réduction classique, pouvant être par exemple une réaction d'hydrogénation par catalyse hétérogène en présence de Pd/C, ou encore une réaction de réduction par le bisulfite de sodium, par certains métaux (notamment le zinc) ou par des boranes (Advanced Organic Chemistry, 4th edition, 1992, J. MARCH, WILEY Interscience ; Reduction in Organic Chemistry, M. Hudlicky, 1983, Ellis Honwood series Chemical Science).
Voie de synthèse 2 : 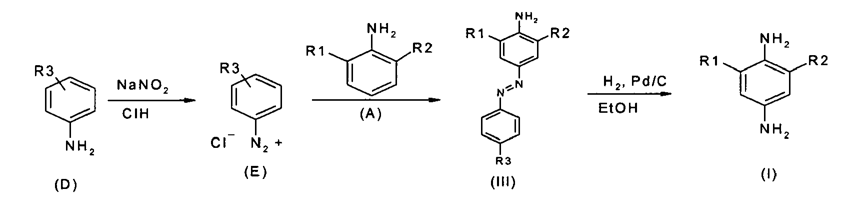 La première étape de la synthèse est la formation d'un azoïque (III) par réaction d'un sel de diazonium (E) avec une aniline (A) selon des méthodes bien connues (Hegarty, in Patai The chemistry of Diazonium and Diazo Group, pt.2 ; Wiley : New York, 1978), le sel de diazonium (E) étant lui-même formé à partir de l'aniline (D). La deuxième étape permettant d'accéder aux composés (I) est une étape de réduction classique, pouvant être par exemple une réaction d'hydrogénation par catalyse hétérogène en présence de Pd/C, ou encore une réaction de réduction par le bisulfite de sodium, par certains métaux (notamment le zinc) ou par des boranes (Advanced Organic Chemistry, 4th edition, 1992, J. MARCH, WILEY Interscience ; Reduction in Organic Chemistry, M. Hudlicky, 1983, Ellis Honwood series Chemical Science).
Voie de synthèse 3 : 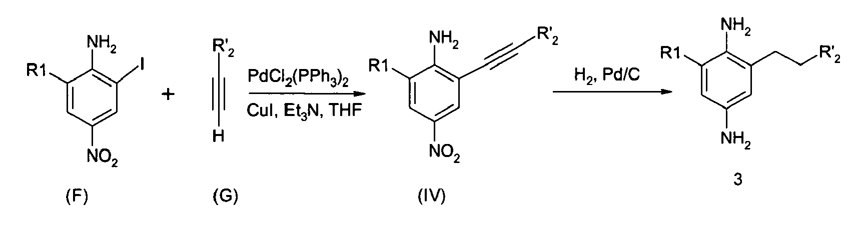 La première étape de la synthèse est une réaction de Sonogashira entre le composé (F) et le composé (G), notamment décrite dans la publication J. Org. Chem. 2003, 68, 3327-3329. La deuxième étape permettant d'accéder aux composés 3 est une étape de réduction classique, pouvant être par exemple une réaction d'hydrogénation par catalyse hétérogène en présence de Pd/C, ou encore une réaction de réduction par le bisulfite de sodium, par certains métaux (notamment le zinc) ou par des boranes (Advanced Organic Chemistry, 4th edition, 1992, J. MARCH, WILEY Interscience ; Reduction in Organic Chemistry, M. Hudlicky, 1983, Ellis Honwood series Chemical Science).
   R₁, R₂, R'₂ et R₃ ayant les significations précédemment énoncées.
   Par « composé intermédiaire de formule (II) ou (III) ou (IV) correspondant, on entend que le radical R₁ dudit composé intermédiaire de formule (II) ou (III) ou (IV) a la même signification que celui du composé (I). Il en est de même pour R₂ dans les composés intermédiaires de formule (II) ou (III).
   Le « composé intermédiaire de formule (III) » portant le groupe alcyne - CC-R'₂ conduit nécessairement après réduction au groupe ―CH₂-CH₂-R'₂.

La composition tinctoriale selon l'invention contient notamment de 0,001 à 10% en poids, de préférence de 0,05 à 6% en poids, et encore plus préférentiellement de 0,1 à 3% en poids, d'au moins une para-phénylènediamine primaire 2,6-substituée de formule (I) ou ses sels ou solvates.

La composition tinctoriale conforme à l'invention peut aussi contenir, en plus du (ou des) para-phénylènediamines primaires 2,6-substituées définie(s) ci-dessus, au moins une base d'oxydation additionnelle qui peut être choisie parmi les bases d'oxydation classiquement utilisées en teinture d'oxydation et parmi lesquelles on peut notamment citer les para-phénylènediamines additionnelles différentes des para-phénylènes primaires 2,6-substituées de formule (I), les bis-phénylalkylènediamines, les para-aminophénols, les ortho-aminophénols et des bases hérétocycliques.

Parmi les para-phénylènediamines additionnelles, on peut plus particulièrement citer à titre d'exemple, la para-phénylènediamine, la para-toluènediamine, la 2,6-diméthyl para-phénylènediamine, la 2-β-hydroxyéthyl para-phénylènediamine, la N-dipropyl para-phénylènediamine, la 2-isopropyl para-phénylènediamine, la N-(β-hydroxypropyl) para-phénylènediamine, la N,N-bis-(β-hydroxyéthyl) para-phénylènediamine, la 4-amino N-(β-méthoxyéthyl) aniline, les para-phénylènediamines décrites dans la demande de brevet français FR 2630438, et leurs sels d'addition.

Parmi les bis-phénylalkylènediamines, on peut plus particulièrement citer à titre d'exemple, le N,N'-bis-(β-hydroxyéthyl) N,N'-bis-(4'-aminophényl) 1,3-diaminopropanol, la N,N'-bis-(β-hydroxyéthyl) N,N'-bis-(4'-aminophényl) éthylène-diamine, la N,N'-bis-(4-amino phényl) tétra-méthylènediamine, la N,N'-bis-(β-hydroxyéthyl), N,N'-bis-(4-aminophényl) tétra-méthylènediamine, la N,N'-bis-(4-méthylaminophényl) tétra-méthylène-diamine, la N,N'-bis-(éthyl) N,N'-bis-(4'-amino, 3'-méthylphényl) éthylènediamine, et leurs sels d'addition.

Parmi les para-aminophénols, on peut plus particulièrement citer à titre d'exemple, le para-aminophénol, le 4-amino 3-méthyl phénol, le 4-amino 3- fluoro phénol, le 4-amino 3-hydroxy-méthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, et leurs sels d'addition.

Parmi les ortho-aminophénols, on peut plus particulièrement citer à titre d'exemple, le 2-amino phénol, le 2-amino 5-méthyl phénol, le 2-amino 6-méthylphénol, le 5-acétamido 2-amino phénol, le 5-[(2-hydroxyéthyl)amino]-2-méthylphénol et leurs sels d'addition.

Parmi les bases hétérocycliques, on peut plus particulièrement citer à titre d'exemple, les dérivés pyridiniques, les dérivés pyrimidiniques, les dérivés pyrazoliques, et leurs sels d'addition.

Lorsqu'elles sont utilisées, ces bases d'oxydation additionnelles représentent de préférence de 0,0005 à 12% en poids du poids total de la composition tinctoriale, et encore plus préférentiellement de 0,005 à 6% en poids de ce poids.

Les compositions de teinture d'oxydation conformes à l'invention peuvent également renfermer au moins un coupleur et/ou au moins un colorant direct, notamment pour modifier les nuances ou les enrichir en reflets.

Les coupleurs utilisables dans les compositions de teinture d'oxydation conformes à l'invention peuvent être choisis parmi les coupleurs utilisés de façon classique en teinture d'oxydation et parmi lesquels on peut notamment citer les méta-phénylènediamines, les méta-aminophénols, les méta-diphénols, les dérivés mono ou polyhydroxylés du naphtalène et les coupleurs hétérocycliques tels que par exemple les dérivés indoliques ou pyridiniques et leurs sels d'addition.

Ces coupleurs sont plus particulièrement choisis parmi le 2-méthyl 5-aminophénol, le 5-N-(β-hydroxyéthyl)amino 2-méthyl phénol, le 6-chloro-2-méthyl-5-aminophénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le 1-β-hydroxyéthylamino-3,4-méthylènedioxybenzène, l'α-naphtol, le 2 méthyl-1-naphtol, le 6-hydroxy indole, le 4-hydroxy indole, le 4-hydroxy N-méthyl indole, la 2-amino-3-hydroxy pyridine, la 6- hydroxy benzomorpholine la 3,5-diamino-2,6-diméthoxypyridine, le 1-N-(β-hydroxyéthyl)amino-3,4-méthylène dioxybenzène, le 2,6-bis-(β-hydroxyéthylamino)toluène et leurs sels d'addition.

Lorsqu'ils sont présents, ces coupleurs représentent notamment de 0,0001 à 10% du poids total de la composition tinctoriale, de préférence de 0,005 à 5% en poids, et encore plus préférentiellement de 0,1 à 3% de ce poids.

D'une manière générale, les sels d'addition avec un acide utilisables dans le cadre des compositions tinctoriales de l'invention (bases d'oxydation et coupleurs) sont notamment choisis parmi les sels des acides suivants : chlorhydrique, bromhydrique, sulfurique, acétique, lactique, tartrique, citrique, méthanesulfonique ou succinique.

Le milieu approprié pour la teinture (ou support) utilisé selon l'invention est constitué par de l'eau ou par un mélange d'eau et d'au moins un solvant organique choisi parmi les alcanols inférieurs en C₁-C₄, les polyols et éthers de polyols, les alcools aromatiques, et leurs mélanges.

La composition tinctoriale selon l'invention peut également contenir divers adjuvants utilisés classiquement dans les compositions pour la teinture des cheveux, tels que des agents tensio-actifs anioniques, cationiques, non-ioniques, amphotères, zwitterioniques ou leurs mélanges, des polymères anioniques, cationiques, non-ioniques, amphotères, zwitterioniques ou leurs mélanges, des agents épaississants minéraux ou organiques, des agents antioxydants, des agents réducteurs, des filtres solaires, des agents de pénétration, des agents séquestrants, des parfums, des tampons, des agents dispersants, des agents de conditionnement tels que par exemple des silicones, des agents filmogènes, des agents conservateurs, des agents opacifiants.

Le pH de la composition tinctoriale selon l'invention est compris entre 3 et 12.

Bien entendu, l'homme de l'art veillera à choisir ce ou ces éventuels composés complémentaires de manière telle que les propriétés avantageuses attachées intrinsèquement à la composition de teinture d'oxydation conforme à l'invention ne soient pas, ou substantiellement pas, altérées par la ou les adjonctions envisagées.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

L'invention a également pour objet un procédé de teinture des fibres kératiniques et en particulier des fibres kératiniques humaines telles que les cheveux mettant en oeuvre la composition tinctoriale telle que définie précédemment.

Selon ce procédé, on applique sur les fibres au moins une composition tinctoriale telle que définie précédemment, pendant un temps suffisant pour développer la coloration désirée, soit à l'air, soit à l'aide d'un agent oxydant. La composition tinctoriale peut éventuellement contenir des catalyseurs d'oxydation, afin d'accélérer le processus d'oxydation.

Selon une première forme de mise en oeuvre du procédé de l'invention, la coloration des fibres peut être effectuée sans addition d'un agent oxydant, au seul contact de l'oxygène de l'air.

Selon une deuxième forme de mise en oeuvre du procédé de l'invention, on applique sur les fibres au moins une composition tinctoriale telle que définie précédemment, la couleur étant révélée à pH acide, neutre ou alcalin à l'aide d'un agent oxydant qui est ajouté juste au moment de l'emploi à la composition tinctoriale ou qui est présent dans une composition oxydante appliquée simultanément ou séquentiellement de façon séparée.

Selon cette deuxième forme de mise en oeuvre du procédé de teinture de l'invention, on mélange de préférence, au moment de l'emploi, la composition tinctoriale décrite ci-dessus avec une composition oxydante contenant, dans un milieu approprié pour la teinture, au moins un agent oxydant présent en une quantité suffisante pour développer une coloration. Le mélange obtenu est ensuite appliqué sur les fibres kératiniques et on laisse poser pendant 3 à 50 minutes, de préférence 5 à 30 minutes, après quoi on rince, on lave au shampooing, on rince à nouveau et on sèche.

L'agent oxydant présent dans la composition oxydante telle que définie ci-dessus peut être choisi parmi les agents oxydants classiquement utilisés pour la teinture d'oxydation des fibres kératiniques, et parmi lesquels on peut citer le peroxyde d'hydrogène, le peroxyde d'urée, les bromates de métaux alcalins, les persels tels que les perborates et persulfates. Le peroxyde d'hydrogène est particulièrement préféré.

Le pH de la composition oxydante renfermant l'agent oxydant tel que défini ci-dessus est tel qu'après mélange avec la composition tinctoriale, le pH de la composition résultante appliquée sur les fibres kératiniques varie de préférence entre 3 et 12, et encore plus préférentiellement entre 5 et 11. Il est ajusté à la valeur désirée au moyen d'agents acidifiants ou alcalinisants habituellement utilisés en teinture des fibres kératiniques et tels que définis précédemment.

La composition oxydante telle que définie ci-dessus peut également renfermer divers adjuvants utilisés classiquement dans les compositions pour la teinture des cheveux et tels que définis précédemment.

La composition qui est finalement appliquée sur les fibres kératiniques peut se présenter sous des formes diverses, telles que sous forme de liquides, de crèmes, de gels, ou sous toute autre forme appropriée pour réaliser une teinture des fibres kératiniques, et notamment des cheveux humains.

Un autre objet de l'invention est un dispositif à plusieurs compartiments ou "kit" de teinture ou tout autre système de conditionnement à plusieurs compartiments dont un premier compartiment renferme la composition tinctoriale telle que définie ci-dessus et un second compartiment renferme la composition oxydante telle que définie ci-dessus. Ces dispositifs peuvent être équipés d'un moyen permettant de délivrer sur les cheveux le mélange souhaité, tel que les dispositifs décrits dans le brevet FR-2 586 913 au nom de la demanderesse.

L'invention porte enfin sur l'utilisation des composés précédemment définis à titre de précurseurs de colorants d'oxydation.

Les exemples qui suivent sont destinés à illustrer l'invention sans toutefois en limiter la portée.

### EXEMPLES

### Mode opératoire voie de synthèse 1

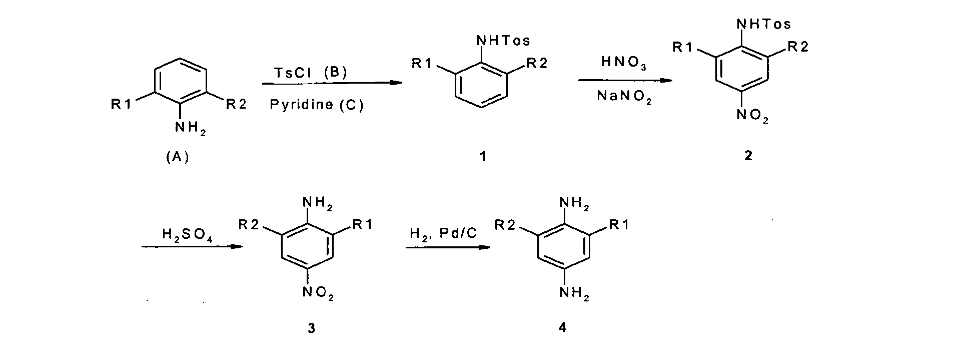

### Synthèse des composés (1)

L'aniline (A), le chlorure d'acide para-toluènesulfonique (B) et la pyridine (C) sont chauffés à reflux pendant une nuit. Le mélange réactionnel est refroidi, on verse sur de la glace et le précipité correspondant aux composés 1 est filtré, lavé à l'eau et séché.

### 1a : N-(2-éthyl-6-méthylphényl)-4-méthylbenzènesulfonamide

Aniline (A): 6-éthyle-o-toluidine : 4.7 g, 1 éq.
Chlorure de tosyle (B) : 7.96 g, 1.2 éq.
Pyridine (C) : 7 ml
Masse obtenue : 9.38 g (rendement : 93 %)
Solide beige
M (m/z) : M⁻=288

### 1b : N-(2-isopropyl-6-méthylphényl)-4-méthylbenzènesulfonamide

Aniline (A) : 2-isopropyl-6-méthylaniline 4.7g, 1 éq.
Chlorure de tosyle (B) : 7.21 g, 1.2 éq.
Pyridine (C ) : 7 ml
Masse obtenue : 8.17 g (rendement : 86 %).
Solide blanc
M (m/z) : M⁻=302

### 1c : N-(2-tert-butyl-6-méthyl-phényl)-4-méthylbenzènesulfonamide

Aniline (A): 6 tert-butyl-o-toluidine : 5 g, 1 éq.
Chlorure de tosyle (B) : 7 g, 1.2 éq.
Pyridine (C) : 10 ml
Masse obtenue : 9.5 g (rendement : 98 %)
Solide beige
M (m/z) : M⁻=316

### Synthèse des composés (2)

L'aniline tosylée 1, l'acide nitrique concentré, l'acide acétique glacial, le nitrite de sodium et l'eau distillée sont chauffés à reflux pendant 3 heures. Le mélange réactionnel est refroidi, et versé sur de la glace. Le précipité correspondant aux composés 2 est filtré et lavé à l'eau et séché sous vide.

### 2a :N-(2-éthyl-6-méthyl-4-nitrophényl)-4-méthylbenzènesulfonamide

Réactif 1a : 2 g, 1 éq.
Acide nitrique : 1.74 ml, 6 éq.
Nitrite de sodium : 0.05 g, 0.1éq.
Eau : 16 ml
Acide acétique glacial : 16 ml
Masse obtenue : 1.66 g (rendement 72 %).
Solide jaune pâle
M(m/z) : M⁻=333

### 2b : N-(2-isopropyl-6-méthyl-4-nitrophényl)-4-méthylbenzène sulfonamide

Réactif 1b : 4 g, 1éq.
Acide nitrique : 3.28 ml, 6 éq.
Nitrite de sodium : 0.09 g, 0.1 éq.
Eau : 25 ml
Acide acétique glacial : 25 ml
Masse obtenue : 5 g (rendement 100 %).
Solide jaune pâle
M(m/z) : M⁻=347

### 2c : N-(2-tert-butyl-6-méthyl-4-nitrophényl)-4-méthylbenzènesulfonamide

Réactif 1c : 5 g, 1éq.
Acide nitrique : 3.92 ml, 6 éq.
Nitrite de sodium : 0.108 g, 0.1éq.
Eau : 30 ml
Acide acétique glacial : 30 ml
Masse obtenue : 5.1 g (rendement 89 %).
Solide beige
M(m/z) : M⁻=361

### Synthèse des composés (3)

Le composé 2 est ajouté à un mélange d'acide sulfurique concentré et d'eau distillée à température ambiante pendant une nuit. Le mélange réactionnel est versé dans de 1a glace puis basifié avec de la soude. Le précipité obtenu correspondant au composé 3 est filtré et lavé à l'eau.

### 3a : 2-éthyl-6-méthyl-4-nitroaniline

Réactif 2a: 1.5 g
Acide sulfurique concentré : 50 ml
Eau distillée : 5 ml
Masse obtenue : 0.78 g (rendement 97 %).
Solide orange
M(m/z) : M⁻=179

### 3b : 2-isopropyl-6-méthyl-4-nitroaniline

Réactif 2b : 5 g
Acide sulfurique concentré : 50 ml
Eau distillée : 5 ml
Masse obtenue : 1.0 g (rendement 37 %)
Solide vert
M(m/z) : M⁻=193

### 3c : 2-tert-butyl-6-méthyl-4-nitroaniline

Réactif 2c: 5 g
Acide sulfurique concentré : 50 ml
Eau distillée : 5 ml
Masse obtenue : 2.5 g (rendement 87 %)
Solide vert
M(m/z) : M⁻=207

### Mode opératoire voie de synthèse 2

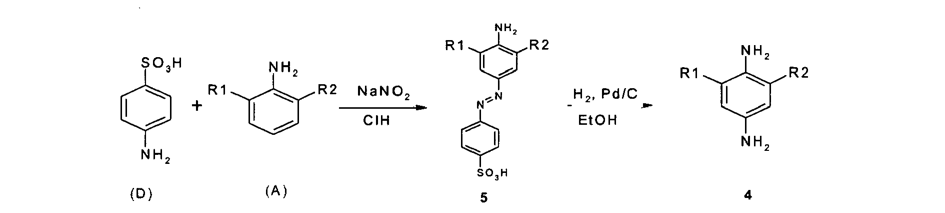

### Synthèse des composés (5)

Une solution de nitrite de sodium dans l'eau est additionnée à froid à l'acide sulfanilique dissout dans l'eau (couleur orange). La température est maintenue inférieure à 10°C.

La solution obtenue est immédiatement versée dans un mélange d'acide chlorhydrique concentré et de glace.

Le sel de diazonium froid est additionné à l'aniline (A) en solution dans l'acide chlorhydrique 0.5 N (une couleur orange sang apparaît ). Le précipité formé correspondant aux composés 5 est filtré et lavé une fois à l'eau.

### 5d Acide 4-[(E)-(4-amino-3-méthoxy-5-méthylphényl)diazényl] benzènesulfonique

Amine (A) : 2-Méthoxy-6-méthylaniline, 6.85 g, 1éq.
HCl (0.5 N) : 100 ml
Acide sulfanilique : 8.66g (1 éq.) dans 50ml d'eau
Nitrite de sodium : 3.73g (1.08 éq.) dans 10 ml d'eau
Acide chlorhydrique 35 % : 12.5 ml (2.5 éq. ) dans 60g de glace
Masse obtenue : 6.1 g (rendement 38 %).
Solide rouge
M(m/z) : M- = 320

### 5e Acide 4-{(E)-(4-amino-3-méthoxy-5-(2-pyrrolidin-1-yléthyl)phényl]diazényl} benzène sulfonique

Amine (A) : 1(2(2-amino-3-méthoxyphényl)éthyl)pyrrolidine, 5 g, 1 éq.
HCl (0.5 N) : 80 ml
Acide sulfanilique : 3.93 g, 1 éq. dans 25 ml d'eau
Nitrite de sodium : 1.69 g, 1.08 éq. dans 5 ml d'eau
Acide chlorhydrique 35 % : 6 ml, 2.5 éq. dans 30 g de glace
Masse obtenue : 2.52 g (rendement 27 %).
Solide rouge
M(m/z) : M⁺= 405

### Etape de Réduction des voies 1 et 2

Dans un hydrogénateur de 0.5 litre, on place le composé 3 ou 5, le catalyseur (palladium sur charbon) et l'éthanol.

La réduction se fait sous une pression d'hydrogène d'environ six bars à une température de 50°C. Après filtration du catalyseur sous azote, on coule sur de l'acide chlorhydrique aqueux. On évapore le filtrat à sec sous pression réduite. Le produit est cristallisé dans l'éthanol chlorhydrique et séché à 40°C sous vide et sur potasse.

### 4a Dichlorhydrate de 2-éthyl-6-méthylbenzène-1,4-diamine

Réactif 3a: 0.8 g
Pd/C : 0.2 g
Ethanol absolu : 250 ml
Masse obtenue : 0.6 g (rendement 91 %).
Solide beige
M(m/z) : M⁺= 151

### 4b Dichlorhydrate de 2-iso-propyl-6-méthylbenzène-1,4-diamine

Réactif 3b: 1 g
Pd/C : 0.3 g
Ethanol absolu : 250 ml
Masse obtenue : 0.82 g (rendement 97 %).
Solide beige
M(m/z) : M⁺= 165

### 4c Dichlorhydrate de 2-tert-butyl-6-méthylbenzène-1,4-diamine

Réactif 3c: 2.2 g
Pd/C : 0.6 g
Ethanol absolu : 250 ml
Masse obtenue : 2.15 g (rendement 81 %).
Solide beige
M(m/z) : M⁺=179

### 4d Dichlorhydrate de 2-méthoxy-6-méthylbenzène-1,4-diamine

Réactif 5d: 6.1 g
Pd/C : 1.2 g
Ethanol absolu : 250 ml
Masse obtenue : 2.91 g (rendement 68 %).
Solide rose
M(m/z) : M⁺=153

### 4e Dichlorhydrate de 2-méthoxy-6-(2-pyrrolidin-1-yléthyl)benzène-1,4-diamine

Réactif 5e: 2.45 g
Pd/C : 0.8 g
Ethanol absolu : 250 ml
Masse obtenue : 0.75 g (rendement 40 %).
Solide vert
M(m/z) : M⁺=236

### 4f Dichlorhydrate d'acide 2,5-diamino-3-méthylbenzoïque

Réactif commercial : acide 2-amino-3-méthyl-5-nitro benzoïque : 2 g
Pd/C : 0.2 g
Ethanol absolu : 250 ml
Masse obtenue : 1.3 g (rendement 70%).
Solide beige
M(m/z) : M⁺ =165

### 4g Dichlorhydrate de 2,6-Diisopropyl-benzène-1,4-diamine

Réactif commercial : 2,6-Diisopropyl-4-nitro-phénylamine : 2g
Pd/C : 0.3g
Ethanol absolu : 250 ml
Masse obtenue : 1.68 g (rendement 66 %).
Solide beige
M(m/z) : M⁺ = 193

### Exemples de formulation

### Exemples 1 à 5 : Composition tinctoriale à partir du Dichlorhydrate d'acide 2,5-diamino-3-méthyl-benzoïque (4f)

### • Exemples 1 à 3 : Teinture en milieu acide

Les compositions tinctoriales suivantes sont préparées :

| Exemple | 1 | 2 | 3 |
|---|---|---|---|
| Dichlorhydrate d'acide 2,5-diamino-3-méthyl-benzoïque | 10-3 mole | 10-3 mole | 10-3 mole |
| 2-Amino-pyridin-3-ol | 10-3 mole | | |
| 2-(2,4-Diamino-phénoxy)-éthanol, chlorhydrate | | 10-3 mole | |
| 3-Amino-2-chloro-6-méthyl-phénol, chlorhydrate | | | 10-3 mole |
| Support de teinture (1) | (*) | (*) | (*) |
| Eau déminéralisée q.s.p. | 100g | 100g | 100g |

| | | | |
|---|---|---|---|
| (*) : support de teinture (1) pH 7 | | | |

| | |
|---|---|
| Alcool éthylique à 96° | 20,8 g |
| Métabisulfite de sodium en solution aqueuse à 35% | 0,23 g M.A |
| Sel pentasodique de l'acide diéthylène-triamine-pentaacétique en solution aqueuse à 40% | 0,48 g M.A |
| Alkyl en C₈-C₁₀ polyglucoside en solution aqueuse à 60% | 3,6 g M.A |
| Alcool benzylique | 2,0 g |
| Polyéthylène glycol à 8 motifs d'oxyde d'éthylène | 3,0 g |
| Na₂HPO₄ | 0,28 g |
| KH₂PO₄ | 0,46 g |

Au moment de l'emploi, chaque composition est mélangée avec un poids égal d'eau oxygénée à 20 volumes (6% en poids). On obtient un pH final de 7.

Chaque mélange obtenu est appliqué sur des mèches de cheveux gris à 90 % de blancs. Après 30 minutes de pose, les mèches sont rincées, lavées avec un shampooing standard, rincées à nouveau puis séchées.

Les nuances obtenues figurent dans le tableau ci-dessous :

| Exemple | 1 | 2 | 3 |
|---|---|---|---|
| Nuance observée | orangé | gris bleu | gris violet-rouge |

### • Exemples 4 à 5 : teinture en milieu basique

Les compositions tinctoriales suivantes sont préparées :

| Exemple | 4 | 5 |
|---|---|---|
| Dichlorhydrate d'acide 2,5-diamino-3-méthyl-benzoïque | 10-3 mole | 10-3 mole |
| 2-(2,4-Diamino-phénoxy)-éthanol, chlorhydrate | 10-3 mole | |
| 3-Amino-2-chloro-6-méthyl-phénol, chlorhydrate | | 10-3 mole |
| Support de teinture (2) | (*) | (*) |
| Eau déminéralisée q.s.p. | 100g | 100g |

| | | |
|---|---|---|
| (*) : support de teinture (2) pH 9.5 | | |

| | |
|---|---|
| Alcool éthylique à 96° | 20,8 g |
| Métabisulfite de sodium en solution aqueuse à 35% | 0,23 g |
| | M.A |
| Sel pentasodique de l'acide diéthylène-triamine-pentaacétique en solution aqueuse à 40% | 0,48 g |
| | M.A |
| Alkyl en C₈-C₁₀ polyglucoside en solution aqueuse à 60% | 3,6 g M.A |
| Alcool benzylique | 2,0 g |
| Polyéthylène glycol à 8 motifs d'oxyde d'éthylène | 3,0 g |
| NH₄Cl | 4,32 g |
| Ammoniaque à 20% de NH₃ | 2,94 g |

Au moment de l'emploi, chaque composition est mélangée avec un poids égal d'eau oxygénée à 20 volumes (6% en poids). On obtient un pH final de 9,5.

Chaque mélange obtenu est appliqué sur des mèches de cheveux gris à 90 % de blancs. Après 30 minutes de pose, les mèches sont rincées, lavées avec un shampooing standard, rincées à nouveau puis séchées.

Les nuances obtenues figurent dans le tableau ci-dessous :

| Exemple | 4 | 5 |
|---|---|---|
| Nuance observée | gris bleu | violet-rouge |

### Exemples 6 à 18 : Composition tinctoriale à partir du Dichlorhydrate de 2,6-diisopropyl-benzène-1,4-diamine (4g)

### • Exemples 6 à 12 : Teinture en milieu acide

Les compositions tinctoriales suivantes sont préparées :

| Exemple | 6 | 7 | 8 | 9 | 10 | 11 | 12 |
|---|---|---|---|---|---|---|---|
| Dichlorhydrate de 2,6-diisopropyl-benzène-1,4-diamine | 10-3 mole | 10-3 mole | 10-3 mole | 10-3 mole | 10-3 mole | 10-3 mole | 10-3 mole |
| Benzène-1,3-diol | 10-3 mole | | | | | | |
| 5-Amino-2-methyl-phénol | | 10-3 mole | | | | | |
| 1H-Indol-6-ol | | | 10-3 mole | | | | |
| 2-Amino-pyridin-3-ol | | | | 10-3 mole | | | |
| 3,6-Diméthyl-1H-pyrazolo[5,1-c][1,2 ,4]triazole | | | | | 10-3 mole | | |
| 2-(2,4-Diamino-phénoxy)-éthanol, chlorhydrate | | | | | | 10-3 mole | |
| 3-Amino-2-chloro-6-méthyl-phénol, chlorhydrate | | | | | | | 10-3 mole |
| Support de teinture (1) | (*) | (*) | (*) | (*) | (*) | (*) | (*) |
| Eau déminéralisée q.s.p. | 100g | 100g | 100g | 100g | 100g | 100g | 100g |

| | | | | | | | |
|---|---|---|---|---|---|---|---|
| (*) : support de teinture (1) pH 7 | | | | | | | |

| | |
|---|---|
| Alcool éthylique à 96° | 20,8 g |
| Métabisulfite de sodium en solution aqueuse à 35% | 0,23 g M.A |
| Sel pentasodique de l'acide diéthylène-triamine-pentaacétique en solution aqueuse à 40% | 0,48 g M.A |
| Alkyl en C₈-C₁₀ polyglucoside en solution aqueuse à 60% | 3,6 g M.A |
| Alcool benzylique | 2,0 g |
| Polyéthylène glycol à 8 motifs d'oxyde d'éthylène | 3,0 g |
| Na₂HPO₄ | 0,28 g |
| KH₂PO₄ | 0,46 g |

Au moment de l'emploi, chaque composition est mélangée avec un poids égal d'eau oxygénée à 20 volumes (6% en poids). On obtient un pH final de 7.

Chaque mélange obtenu est appliqué sur des mèches de cheveux gris à 90 % de blancs. Après 30 minutes de pose, les mèches sont rincées, lavées avec un shampooing standard, rincées à nouveau puis séchées.

Les nuances obtenues figurent dans le tableau ci-dessous :

| Exemple | 6 | 7 | 8 | 9 | 10 | 11 | 12 |
|---|---|---|---|---|---|---|---|
| Nuance observée | jaune | rouge intense | rouge intense | rouge intense | rouge | bleu intense | violet intense |

### • Exemples 13 à 18 : teinture en milieu basique

Les compositions tinctoriales suivantes sont préparées :

| Exemple | 13 | 14 | 15 | 16 | 17 | 18 |
|---|---|---|---|---|---|---|
| Dichlorhydrate de 2,6-diisopropyl-benzène-1,4-diamine | 10-3 mole | 10-3 mole | 10-3 mole | 10-3 mole | 10-3 mole | 10-3 mole |
| 5-Amino-2-methyl-phénol | 10-3 mole | | | | | |
| 1H-Indol-6-ol | | 10-3 mole | | | | |
| 2-Amino-pyridin-3-ol | | | 10-3 mole | | | |
| 3,6-Diméthyl-1H-pyrazolo[5,1-c][1,2,4]triazole | | | | 10-3 mole | | |
| 2-(2,4-Diamino-phénoxy)-éthanol,chlorhydrate | | | | | 10-3 mole | |
| 3-Amino-2-chloro-6-méthyl-phénol,chlorhydrate | | | | | | 10-3 mole |
| Support de teinture (2) | (*) | (*) | (*) | (*) | (*) | (*) |
| Eau q.s.p. | déminéralisée | 100g | 100g | 100g | 100g | 100g |

| | | | | | | |
|---|---|---|---|---|---|---|
| (*) : support de teinture (2) pH 9.5 | | | | | | |

| | |
|---|---|
| Alcool éthylique à 96° | 20,8 g |
| Métabisulfite de sodium en solution aqueuse à 35% | 0,23 g |
| | M.A |
| Sel pentasodique de l'acide diéthylène-triamine- | 0,48 g |
| pentaacétique en solution aqueuse à 40% | M.A |
| Alkyl en C₈-C₁₀ polyglucoside en solution aqueuse à 60% | 3,6 g M.A |
| Alcool benzylique | 2,0 g |
| Polyéthylène glycol à 8 motifs d'oxyde d'éthylène | 3,0 g |
| NH₄Cl | 4,32 g |
| Ammoniaque à 20% de NH₃ | 2,94 g |

Au moment de l'emploi, chaque composition est mélangée avec un poids égal d'eau oxygénée à 20 volumes (6% en poids). On obtient un pH final de 9,5.

Chaque mélange obtenu est appliqué sur des mèches de cheveux gris à 90 % de blancs. Après 30 minutes de pose, les mèches sont rincées, lavées avec un shampooing standard, rincées à nouveau puis séchées.

Les nuances obtenues figurent dans le tableau ci-dessous :

| Exemple | 13 | 14 | 15 | 16 | 17 | 18 |
|---|---|---|---|---|---|---|
| Nuance observée | rouge | rouge | rouge | rouge chromatique | bleu intense | violet intense |

### Exemples 19 à 31 : Composition tinctoriale à partir du Dichlorhydrate de 2-éthyl-6-méthyl-benzène-1,4-diamine (4a)

### • Exemples 19 à 25 : Teinture en milieu acide

Les compositions tinctoriales suivantes sont préparées :

| Exemple | 19 | 20 | 21 | 22 | 23 | 24 | 25 |
|---|---|---|---|---|---|---|---|
| Dichlorhydrate de 2-éthyl-6-méthyl-benzène-1,4-diamine | 10-3 mole | 10-3 mole | 10-3 mole | 10-3 mole | 10-3 mole | 10-3 mole | 10-3 mole |
| Benzène-1,3-diol | 10-3 mole | | | | | | |
| 5-Amino-2-méthyl-phénol | | 10-3 mole | | | | | |
| 1H-Indol-6-ol | | | 10-3 mole | | | | |
| 2-Amino-pyridin-3-ol | | | | 10-3 mole | | | |
| 3,6-Diméthyl-1H-pyrazolo[5,1-c][1,2 ,4]triazole | | | | | 10-3 mole | | |
| 2-(2,4-Diamino-phénoxy)-éthanol,chlorhydrat e | | | | | | 10-3 mole | |
| 3-Amino-2-chloro-6-méthyl-phénol,chlorhydrat e | | | | | | | 10-3 mole |
| Support de teinture (1) | (*) | (*) | (*) | (*) | (*) | (*) | (*) |
| Eau déminéralisée q.s.p. | 100g | 100g | 100g | 100g | 100g | 100g | 100g |

| | | | | | | | |
|---|---|---|---|---|---|---|---|
| (*) : support de teinture (1) pH 7 | | | | | | | |

| | |
|---|---|
| Alcool éthylique à 96° | 20,8 g |
| Métabisulfite de sodium en solution aqueuse à 35% | 0,23 g M.A |
| Sel pentasodique de l'acide diéthylène-triamine-pentaacétique en solution aqueuse à 40% | 0,48 g M.A |
| Alkyl en C₈-C₁₀ polyglucoside en solution aqueuse à 60% | 3,6 g M.A |
| Alcool benzylique | 2,0 g |
| Polyéthylène glycol à 8 motifs d'oxyde d'éthylène | 3,0 g |
| Na₂HPO₄ | 0,28 g |
| KH₂PO₄ | 0,46 g |

Au moment de l'emploi, chaque composition est mélangée avec un poids égal d'eau oxygénée à 20 volumes (6% en poids). On obtient un pH final de 7.

Chaque mélange obtenu est appliqué sur des mèches de cheveux gris à 90 % de blancs. Après 30 minutes de pose, les mèches sont rincées, lavées avec un shampooing standard, rincées à nouveau puis séchées.

Les nuances obtenues figurent dans le tableau ci-dessous :

| Exemple | 19 | 20 | 21 | 22 | 23 | 24 | 25 |
|---|---|---|---|---|---|---|---|
| Nuance observée | brun jaune | violet-rouge intense | rouge intense | brun rouge intense | rouge | violet-bleu intense | violet intense |

### • Exemples 26 à 31 : teinture en milieu basique

Les compositions tinctoriales suivantes sont préparées :

| Exemple | 26 | 27 | 28 | 29 | 30 | 31 |
|---|---|---|---|---|---|---|
| Dichlorhydrate de 2-éthyl-6-méthyl-benzène-1,4-diamine | 10-3 mole | 10-3 mole | 10-3 mole | 10-3 mole | 10-3 mole | 10-3 mole |
| 5-Amino-2-méthyl-phénol | 10-3 mole | | | | | |
| 1H-Indol-6-ol | | 10-3 mole | | | | |
| 2-Amino-pyridin-3-ol | | | 10-3 mole | | | |
| 3,6-Diméthyl-1H-pyrazolo[5,1-c][1,2,4]triazole | | | | 10-3 mole | | |
| 2-(2,4-Diamino-phenoxy)-ethanol, chlorhydrate | | | | | 10-3 mole | |
| 3-Amino-2-chloro-6-méthyl-phénol, chlorhydrate | | | | | | 10-3 mole |
| Support de teinture (2) | (*) | (*) | (*) | (*) | (*) | (*) |
| Eau déminéralisée q.s.p. | 100g | 100g | 100g | 100g | 100g | 100g |

| | | | | | | |
|---|---|---|---|---|---|---|
| (*) : support de teinture (2) pH 9.5 | | | | | | |

| | |
|---|---|
| Alcool éthylique à 96° | 20,8 g |
| Métabisulfite de sodium en solution aqueuse à 35% | 0,23 g |
| | M.A |
| Sel pentasodique de l'acide diéthylène-triamine- | 0,48 g |
| pentaacétique en solution aqueuse à 40% | M.A |
| Alkyl en C₈-C₁₀ polyglucoside en solution aqueuse à 60% | 3,6 g M.A |
| Alcool benzylique | 2,0 g |
| Polyéthylène glycol à 8 motifs d'oxyde d'éthylène | 3,0 g |
| NH₄Cl | 4,32 g |
| Ammoniaque à 20% de NH₃ | 2,94 g |

Au moment de l'emploi, chaque composition est mélangée avec un poids égal d'eau oxygénée à 20 volumes (6% en poids). On obtient un pH final de 9,5.

Chaque mélange obtenu est appliqué sur des mèches de cheveux gris à 90 % de blancs. Après 30 minutes de pose, les mèches sont rincées, lavées avec un shampooing standard, rincées à nouveau puis séchées.

Les nuances obtenues figurent dans le tableau ci-dessous :

| Exemple | 26 | 27 | 28 | 29 | 30 | 31 |
|---|---|---|---|---|---|---|
| Nuance observée | rouge | orangé | rouge | rouge chromatique | bleu intense | violet intense |

### Exemples 32 à 44 : Composition tinctoriale à partir du Dichlorhydrate de 2-isopropyl-6-méthyl-benzène-1,4-diamine (4b)

### • Exemples 32 à 38 : Teinture en milieu acide

Les compositions tinctoriales suivantes sont préparées :

| Exemple | 32 | 33 | 34 | 35 | 36 | 37 | 38 |
|---|---|---|---|---|---|---|---|
| Dichlorhydrate de 2-isopropyl-6-méthyl-benzène-1,4-diamine | 10-3 mole | 10-3 mole | 10-3 mole | 10-3 mole | 10-3 mole | 10-3 mole | 10-3 mole |
| Benzène-1,3-diol | 10-3 mole | | | | | | |
| 5-Amino-2-méthyl-phénol | | 10-3 mole | | | | | |
| 1H-Indol-6-ol | | | 10-3 mole | | | | |
| 2-Amino-pyridin-3-ol | | | | 10-3 mole | | | |
| 3,6-Diméthyl-1H-pyrazolo[5,1-c][1,2 ,4]triazole | | | | | 10-3 mole | | |
| 2-(2,4-Diamino-phénoxy)-éthanol, chlorhydrate | | | | | | 10-3 mole | |
| 3-Amino-2-chloro-6-méthyl-phénol, chlorhydrate | | | | | | | 10-3 mole |
| Support de teinture (1) | (*) | (*) | (*) | (*) | (*) | (*) | (*) |
| Eau déminéralisée q.s.p. | 100g | 100g | 100g | 100g | 100g | 100g | 100g |

| | | | | | | | |
|---|---|---|---|---|---|---|---|
| (*) : support de teinture (1) pH 7 | | | | | | | |

| | |
|---|---|
| Alcool éthylique à 96° | 20,8 g |
| Métabisulfite de sodium en solution aqueuse à 35% | 0,23 g M.A |
| Sel pentasodique de l'acide diéthylène-triamine-pentaacétique en solution aqueuse à 40% | 0,48 g M.A |
| Alkyl en C₈-C₁₀ polyglucoside en solution aqueuse à 60% | 3,6 g M.A |
| Alcool benzylique | 2,0 g |
| Polyéthylène glycol à 8 motifs d'oxyde d'éthylène | 3,0 g |
| Na₂HPO₄ | 0,28 g |
| KH₂PO₄ | 0,46 g |

Au moment de l'emploi, chaque composition est mélangée avec un poids égal d'eau oxygénée à 20 volumes (6% en poids). On obtient un pH final de 7.

Chaque mélange obtenu est appliqué sur des mèches de cheveux gris à 90 % de blancs. Après 30 minutes de pose, les mèches sont rincées, lavées avec un shampooing standard, rincées à nouveau puis séchées.

Les nuances obtenues figurent dans le tableau ci-dessous :

| Exemple | 32 | 33 | 34 | 35 | 36 | 37 | 38 |
|---|---|---|---|---|---|---|---|
| Nuance observée | brun jaune | rouge intense | orangé | brun rouge intense | rouge | bleu intense | violet intense |

### • Exemples 39 à 44 : teinture en milieu basique

Les compositions tinctoriales suivantes sont préparées :

| Exemple | 39 | 40 | 41 | 42 | 43 | 44 |
|---|---|---|---|---|---|---|
| Dichlorhydrate de 2-isopropyl-6-méthyl-benzène-1,4-dianune | 10-3 mole | 10-3 mole | 10-3 mole | 10-3 mole | 10-3 mole | 10-3 mole |
| 5-Amino-2-méthyl-phénol | 10-3 mole | | | | | |
| 1H-Indol-6-ol | | 10-3 mole | | | | |
| 2-Amino-pyridin-3-ol | | | 10-3 mole | | | |
| 3,6-Diméthyl-1H-pyrazolo[5,1-c][1,2 ,4]triazole | | | | 10-3 mole | | |
| 2-(2,4-Diamino-phénoxy)-éthanol, chlorhydrate | | | | | 10-3 mole | |
| 3-Amino-2-chloro-6-méthyl-phénol, chlorhydrate | | | | | | 10-3 mole |
| Support de teinture (2) | (*) | (*) | (*) | (*) | (*) | (*) |
| Eau déminéralisée q.s.p. | 100g | 100g | 100g | 100g | 100g | 100g |

| | | | | | | |
|---|---|---|---|---|---|---|
| (*) : support de teinture (2) pH 9.5 | | | | | | |

| | |
|---|---|
| Alcool éthylique à 96° | 20,8 g |
| Métabisulfite de sodium en solution aqueuse à 35% | 0,23 g |
| | M.A |
| Sel pentasodique de l'acide diéthylène-triamine- | 0,48 g |
| pentaacétique en solution aqueuse à 40% | M.A |
| Alkyl en C₈-C₁₀ polyglucoside en solution aqueuse à 60% | 3,6 g M.A |
| Alcool benzylique | 2,0 g |
| Polyéthylène glycol à 8 motifs d'oxyde d'éthylène | 3,0 g |
| NH₄Cl | 4,32 g |
| Ammoniaque à 20% de NH₃ | 2,94 g |

Au moment de l'emploi, chaque composition est mélangée avec un poids égal d'eau oxygénée à 20 volumes (6% en poids). On obtient un pH final de 9,5.

Chaque mélange obtenu est appliqué sur des mèches de cheveux gris à 90 % de blancs. Après 30 minutes de pose, les mèches sont rincées, lavées avec un shampooing standard, rincées à nouveau puis séchées.

Les nuances obtenues figurent dans le tableau ci-dessous :

| Exemple | 39 | 40 | 41 | 42 | 43 | 44 |
|---|---|---|---|---|---|---|
| Nuance observée | rouge | orangé | rouge | rouge chromatique | bleu intense | violet intense |

### Exemples 45 à 57 : Composition tinctoriale à partir du Dichlorhydrate de 2-tert-butyl-6-méthyl-benzène-1,4-diamine (4c)

### • Exemples 45 à 51 : Teinture en milieu acide

| Exemple | 45 | 46 | 47 | 48 | 49 | 50 | 51 |
|---|---|---|---|---|---|---|---|
| Dichlorhydrate de 2-tert-butyl-6-méthyl-benzène-1,4-diamine | 10-3 mole | 10-3 mole | 10-3 mole | 10-3 mole | 10-3 mole | 10-3 mole | 10-3 mole |
| Benzène-1,3-diol | 10-3 mole | | | | | | |
| 5-Amino-2-méthyl-phénol | | 10-3 mole | | | | | |
| 1H-Indol-6-ol | | | 10-3 mole | | | | |
| 2-Amino-pyridin-3-ol | | | | 10-3 mole | | | |
| 3,6-Diméthyl-1H-pyrazolo[5,1-c][1,2,4]triazole | | | | | 10-3 mole | | |
| 2-(2,4-Diamino-phénoxy)-éthanol, chlorhydrate | | | | | | 10-3 mole | |
| 3-Amino-2-chloro-6-méthyl-phénol, chlorhydrate | | | | | | | 10-3 mole |
| Support de teinture (1) | (*) | (*) | (*) | (*) | (*) | (*) | (*) |
| Eau déminéralisée q.s.p. | 100g | 100g | 100g | 100g | 100g | 100g | 100g |

| | | | | | | | |
|---|---|---|---|---|---|---|---|
| (*) : support de teinture (1) pH 7 | | | | | | | |

| | |
|---|---|
| Alcool éthylique à 96° | 20,8 g |
| Métabisulfite de sodium en solution aqueuse à 35% | 0,23 g M.A |
| Sel pentasodique de l'acide diéthylène-triamine-pentaacétique en solution aqueuse à 40% | 0,48 g M.A |
| Alkyl en C₈-C₁₀ polyglucoside en solution aqueuse à 60% | 3,6 g M.A |
| Alcool benzylique | 2,0 g |
| Polyéthylène glycol à 8 motifs d'oxyde d'éthylène | 3,0 g |
| Na₂HPO₄ | 0,28 g |
| KH₂PO₄ | 0,46 g |

Au moment de l'emploi, chaque composition est mélangée avec un poids égal d'eau oxygénée à 20 volumes (6% en poids). On obtient un pH final de 7.

Chaque mélange obtenu est appliqué sur des mèches de cheveux gris à 90 % de blancs. Après 30 minutes de pose, les mèches sont rincées, lavées avec un shampooing standard, rincées à nouveau puis séchées.

Les nuances obtenues figurent dans le tableau ci-dessous :

| Exemple Nuance | 45 | 46 | 47 | 48 | 49 | 50 | 51 |
|---|---|---|---|---|---|---|---|
| Nuance observée | Brun orangé | Violet-rouge intense | Rouge intense | Rouge intense | Rouge | Bleu intense | Violet intense |

### • Exemples 52 à 57 : teinture en milieu basique

Les compositions tinctoriales suivantes sont préparées :

| Exemple | 52 | 53 | 54 | 55 | 56 | 57 |
|---|---|---|---|---|---|---|
| Dichlorhydrate de 2-tert-butyl-6-méthyl-benzène-1,4-diamine | 10-3 mole | 10-3 mole | 10-3 mole | 10-3 mole | 10-3 mole | 10-3 mole |
| 5-Amino-2-méthyl-phénol | 10-3 mole | | | | | |
| 1H-Indol-6-ol | | 10-3 mole | | | | |
| 2-Amino-pyridin-3-ol | | | 10-3 mole | | | |
| 3,6-Diméthyl-1H-pyrazolo[5,1-c][1,2,4]triazole | | | | 10-3 mole | | |
| 2-(2,4-Diamino-phénoxy)-éthanol, chlorhydrate | | | | | 10-3 mole | |
| 3-Amino-2-chloro-6-méthyl-phénol, chlorhydrate | | | | | | 10-3 mole |
| Support de teinture (2) | (*) | (*) | (*) | (*) | (*) | (*) |
| Eau déminéralisée q.s.p. | 100g | 100g | 100g | 100g | 100g | 100g |

| | | | | | | |
|---|---|---|---|---|---|---|
| (*) : support de teinture (2) pH 9.5 | | | | | | |

| | |
|---|---|
| Alcool éthylique à 96° | 20,8 g |
| Métabisulfite de sodium en solution aqueuse à 35% | 0,23 g M.A |
| Sel pentasodique de l'acide diéthylène-triamine-pentaacétique en solution aqueuse à 40% | 0,48 g M.A |
| Alkyle en C₈-C₁₀ polyglucoside en solution | 3,6 g M.A |
| aqueuse à 60% | |
| Alcool benzylique | 2,0 g |
| Polyéthylène glycol à 8 motifs d'oxyde d'éthylène | 3,0 g |
| NH₄Cl | 4,32 g |
| Ammoniaque à 20% de NH₃ | 2,94 g |

Au moment de l'emploi, chaque composition est mélangée avec un poids égal d'eau oxygénée à 20 volumes (6% en poids). On obtient un pH final de 9,5.

Chaque mélange obtenu est appliqué sur des mèches de cheveux gris à 90 % de blancs. Après 30 minutes de pose, les mèches sont rincées, lavées avec un shampooing standard, rincées à nouveau puis séchées.

Les nuances obtenues figurent dans le tableau ci-dessous :

| Exemple | 52 | 53 | 54 | 55 | 56 | 57 |
|---|---|---|---|---|---|---|
| Nuance observée | rouge | rouge | rouge | Rouge chromati que | Bleu intense | Violet intense |

### Exemples 58 à 64 : Composition tinctoriale à partir du Dichlorhydrate de 2-méthoxy-6-méthyl-benzène-1,4-diamine (4d)

### • Exemples 58 à 62 : Teinture en milieu acide

| Exemples | 58 | 59 | 60 | 61 | 62 |
|---|---|---|---|---|---|
| Dichlorhydrate de 2-méthoxy-6-méthyl-benzène-1,4-diamine | 10-3 mole | 10-3 mole | 10-3 mole | 10-3 mole | 10-3 mole |
| 5-Amino-2-méthyl-phénol | 10-3 mole | | | | |
| 1H-Indol-6-ol | | 10-3 mole | | | |
| 2-Amino-pyridin-3-ol | | | 10-3 mole | | |
| 2-(2,4-Diamino-phénoxy)-éthanol,chlorhydrate | | | | 10-3 mole | |
| 3-Amino-2-chloro-6-méthyl-phénol,chlorhydrate | | | | | 10-3 mole |
| Support de teinture (2) | (*) | (*) | (*) | (*) | (*) |
| Eau déminéralisée q.s.p. | 100g | 100g | 100g | 100g | 100g |

| | | | | | |
|---|---|---|---|---|---|
| (*) : support de teinture (1) pH 7 | | | | | |

| | |
|---|---|
| Alcool éthylique à 96° | 20,8 g |
| Métabisulfite de sodium en solution aqueuse à 35% | 0,23 g M.A |
| Sel pentasodique de l'acide diéthylène-triamine-pentaacétique en solution aqueuse à 40% | 0,48 g M.A |
| Alkyl en C₈-C₁₀ polyglucoside en solution aqueuse à 60% | 3,6 g M.A |
| Alcool benzylique | 2,0 g |
| Polyéthylène glycol à 8 motifs d'oxyde d'éthylène | 3,0 g |
| Na₂HPO₄ | 0,28 g |
| KH₂PO₄ | 0,46 g |

Au moment de l'emploi, chaque composition est mélangée avec un poids égal d'eau oxygénée à 20 volumes (6% en poids). On obtient un pH final de 7.

Chaque mélange obtenu est appliqué sur des mèches de cheveux gris à 90 % de blancs. Après 30 minutes de pose, les mèches sont rincées, lavées avec un shampooing standard, rincées à nouveau puis séchées.

Les nuances obtenues figurent dans le tableau ci-dessous :

| Exemple | 58 | 59 | 60 | 61 | 62 |
|---|---|---|---|---|---|
| Nuance observée | brun rouge | rouge intense | brun rouge | gris bleu intense | gris violet-bleu intense |

### • Exemples 63 à 64 : teinture en milieu basique

Les compositions tinctoriales suivantes sont préparées :

| Exemple | 63 | 64 |
|---|---|---|
| Dichlorhydrate de 2-méthoxy-6-méthyl-benzène-1,4-diamine | 10-3 mole | 10-3 mole |
| 5-Amino-2-méthyl-phénol | 10-3 mole | |
| 3-Amino-2-chloro-6-méthyl-phénol,chlorhydrate | | 10-3 mole |
| Support de teinture (1) | (*) | (*) |
| Eau déminéralisée q.s.p. | 100g | 100g |

| | | |
|---|---|---|
| (*) : support de teinture (2) pH 9.5 | | |

| | |
|---|---|
| Alcool éthylique à 96° | 20,8 g |
| Métabisulfite de sodium en solution aqueuse à 35% | 0,23 g M.A |
| Sel pentasodique de l'acide diéthylène-triamine-pentaacétique en solution aqueuse à 40% | 0,48 g M.A |
| Alkyle en C₈-C₁₀ polyglucoside en solution aqueuse à 60% | 3,6 g M.A |
| Alcool benzylique | 2,0 g |
| Polyéthylène glycol à 8 motifs d'oxyde d'éthylène | 3,0 g |
| NH₄Cl | 4,32 g |
| Ammoniaque à 20% de NH₃ | 2,94 g |

Au moment de l'emploi, chaque composition est mélangée avec un poids égal d'eau oxygénée à 20 volumes (6% en poids). On obtient un pH final de 9,5.

Chaque mélange obtenu est appliqué sur des mèches de cheveux gris à 90 % de blancs. Après 30 minutes de pose, les mèches sont rincées, lavées avec un shampooing standard, rincées à nouveau puis séchées.

Les nuances obtenues figurent dans le tableau ci-dessous :

| Exemple | 63 | 64 |
|---|---|---|
| Nuance observée | gris bleu | gris violet-bleu |

### Exemples 65 à 72 : Composition tinctoriale à partir du Dichlorhydrate de 2-méthoxy-6-(2-pyrrolidin-1-yl-éthyl)-benzène-1,4-diamine (4e)

### • Exemples 65 à 69 : Teinture en milieu acide

| Exemple | 65 | 66 | 67 | 68 | 69 |
|---|---|---|---|---|---|
| Dichlorhydrate de_2-méthoxy-6-(2-pyrrolidin-1-yl-éthyl)-benzène-1,4-diamine | 10-3 mole | 10-3 mole | 10-3 mole | 10-3 mole | 10-3 mole |
| 5-Amino-2-methyl-phenol | 10-3 mole | | | | |
| 1H-Indol-6-ol | | 10-3 mole | | | |
| 2-Amino-pyridin-3-ol | | | 10-3 mole | | |
| 2-(2,4-Diamino-phénoxy)-éthanol,chlorhydrate | | | | 10-3 mole | |
| 3-Amino-2-chloro-6-méthyl-phénol,chlorhydrate | | | | | 10-3 mole |
| Support de teinture (2) | (*) | (*) | (*) | (*) | (*) |
| Eau déminéralisée q.s.p. | 100g | 100g | 100g | 100g | 100g |

| | | | | | |
|---|---|---|---|---|---|
| (*) : support de teinture (1) pH 7 | | | | | |

| | |
|---|---|
| Alcool éthylique à 96° | 20,8 g |
| Métabisulfite de sodium en solution aqueuse à 35% | 0,23 g M.A |
| Sel pentasodique de l'acide diéthylène-triamine-pentaacétique en solution aqueuse à 40% | 0,48 g M.A |
| Alkyl en C₈-C₁₀ polyglucoside en solution aqueuse à 60% | 3,6 g M.A |
| Alcool benzylique | 2,0 g |
| Polyéthylène glycol à 8 motifs d'oxyde d'éthylène | 3,0 g |
| Na₂HPO₄ | 0,28 g |
| KH₂PO₄ | 0,46 g |

Au moment de l'emploi, chaque composition est mélangée avec un poids égal d'eau oxygénée à 20 volumes (6% en poids). On obtient un pH final de 7.

Chaque mélange obtenu est appliqué sur des mèches de cheveux gris à 90 % de blancs. Après 30 minutes de pose, les mèches sont rincées, lavées avec un shampooing standard, rincées à nouveau puis séchées.

Les nuances obtenues figurent dans le tableau ci-dessous :

| Exemple | 65 | 66 | 67 | 68 | 69 |
|---|---|---|---|---|---|
| Nuance observée | brun rouge | rouge | rouge gris | bleu intense | gris violet |

### • Exemples 70 à 72 : teinture en milieu basique

Les compositions tinctoriales suivantes sont préparées :

| Exemple | 70 | 71 | 72 |
|---|---|---|---|
| Dichlorhydrate de 2-méthoxy-6-(2-pyrrolidin-1-yl-éthyl)-benzène-1,4-diamine | 10-3 mole | 10-3 mole | 10-3 mole |
| 5-Amino-2-methyl-phenol | 10-3 mole | | |
| 2-(2,4-Diamino-phenoxy)-ethanol,chlorhydrate | | 10-3 mole | |
| 3-Amino-2-chloro-6-methyl-phenol,chlorhydrate | | | 10-3 mole |
| Support de teinture (1) | (*) | (*) | (*) |
| Eau déminéralisée q.s.p. | 100g | 100g | 100g |

| | | | |
|---|---|---|---|
| (*) : support de teinture (2) pH 9.5 | | | |

| | |
|---|---|
| Alcool éthylique à 96° | 20,8 g |
| Métabisulfite de sodium en solution aqueuse | 0,23 g M.A |
| à 35% | |
| Sel pentasodique de l'acide diéthylène-triamine-pentaacétique en solution aqueuse à 40% | 0,48 g M.A |
| Alkyle en C₈-C₁₀ polyglucoside en solution aqueuse à 60% | 3,6 g M.A |
| Alcool benzylique | 2,0 g |
| Polyéthylène glycol à 8 motifs d'oxyde d'éthylène | 3,0 g |
| NH₄Cl | 4,32 g |
| Ammoniaque à 20% de NH₃ | 2,94 g |

Au moment de l'emploi, chaque composition est mélangée avec un poids égal d'eau oxygénée à 20 volumes (6% en poids). On obtient un pH final de 9,5.

Chaque mélange obtenu est appliqué sur des mèches de cheveux gris à 90 % de blancs. Après 30 minutes de pose, les mèches sont rincées, lavées avec un shampooing standard, rincées à nouveau puis séchées.

Les nuances obtenues figurent dans le tableau ci-dessous :

| Exemple | 70 | 71 | 72 |
|---|---|---|---|
| Nuance observée | brun rouge | brun | gris violet |

## Revendications

1. Composé para-phénylènediamine de formule (I), ses solvates et sels d'acide physiologiquement acceptables : 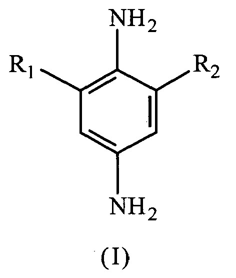 dans laquelle R₁ et R₂ représentent, indépendamment,
- un radical alkyle linéaire ou ramifié en C₁-C₆ éventuellement substitué par un radical alcoxy en C₁-C₆, hydroxy, amino, alkyle(C₁-C₆)amino, dialkyl(C₁-C₆)amino ou encore par un radical cyclique de type :1-pyrrolidinyle, 1-cyclohexylaminyle, 1-pipérazinyle, 1-diazépanyle, 1-morpholinyle, 1-imidazoyle, ce cycle pouvant être substitué par un ou plusieurs groupements alkyle, hydroxy ou amino ;
- un radical alcoxy, linéaire ou ramifié, en C₁-C₆, monohydroxyalcoxy en C₁-C₆, polyhydroxyalcoxy en C₁-C₆;
- un radical carboxylique
- un radical amide.
à l'exception des composés suivants :

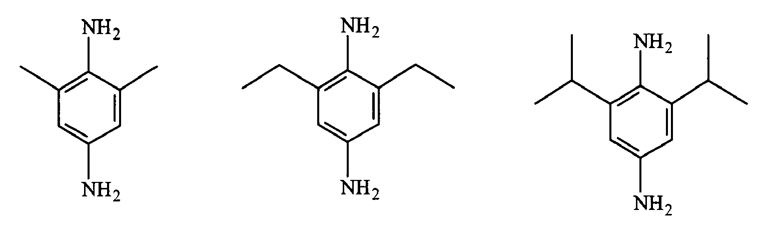

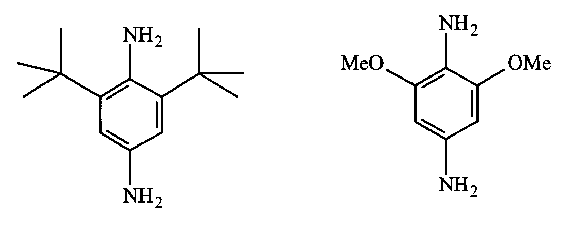

2. Composé de formule (I) selon la revendication 1, **caractérisé par le fait que** R₁ et R₂ représentent indépendamment
- un radical méthyle, éthyle, n-propyle, iso-propyle, sec-butyle, iso-butyle, tert-butyle, (2,2'diméthyl)butyle ;
- un radical méthoxybutyle, aminométhyle, (3-diméthylamino)propyle, imidazolo-n-propyle, pyrrolidinoéthyle ;
- un radical méthoxy, hydroxyméthyle, α-hydroxyéthyle, β-hydroxyéthyle, β-hydroxy-iso-propyle ;
- un radical carboxylique ;
- un radical amido.

3. Composé de formule (I) selon la revendication 1 ou 2 **caractérisé par le fait qu'**il est choisi parmi les composés suivants ou l'un de leurs sels ou solvates physiologiquement acceptables :
| | |
|---|---|
| 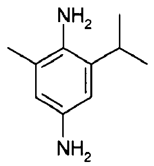 | 2-Isopropyl-6-méthyl-benzène-1,4-diamine |
| 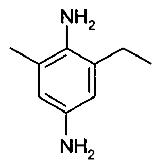 | 2-Ethyl-6-méthyl-benzène-1,4-diamine |
| 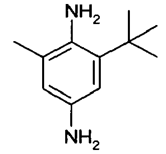 | 2-tert-Butyl-6-méthyl-benzène-1,4-diamine |
| 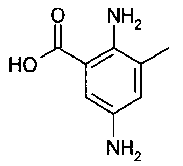 | Acide 2,5-diamino-3-méthyl-benzoïque |
| 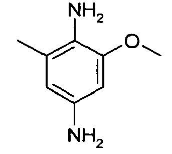 | 2-Méthoxy-6-méthyl-benzène-1,4-diamine |
| 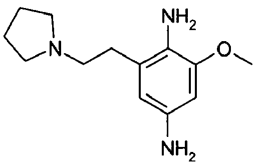 | 2-Méthoxy-6-(2-pyrrolidin-1-yl-éthyl)-benzène-1,4-diamine |
| 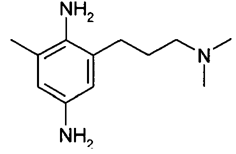 | 2-(3-Diméthylamino-propyl)-6-méthyl -benzène-1,4-diamine |
| 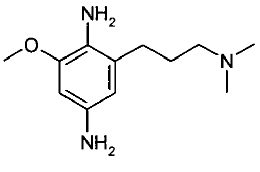 | 2-(3-Diméthylamino-propyl)-6-méthoxy-benzène-1,4-diamine |
| 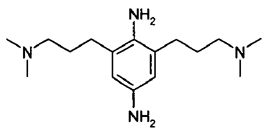 | 2,6-Bis-(3-diméthylamino-propyl)-benzène-1,4-diamine |
| 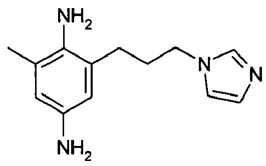 | 2-(3-Imidazol-1-yl-propyl)-6-methyl -benzene-1,4-diamine |
| 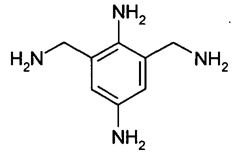 | 2,6-Bis-aminométhyl-benzène-1,4-diamine |
| 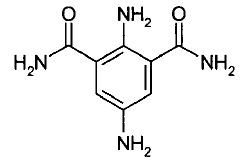 | 2,5-Diamino-isophthalamide |
| 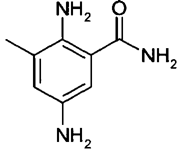 | 2,5-Diamino-3-méthylbenzamide |
| 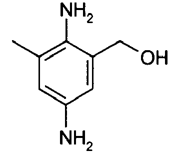 | (2,5-Diamino-3-méthyl-phényl)-méthanol |
| 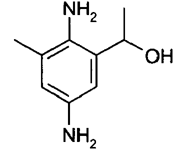 | 1-(2,5-Diamino-3-méthyl-phényl)-éthanol |
| 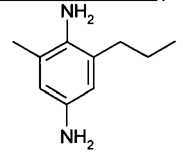 | 2-Méthyl-6-propyl-benzène-1,4-diamine |
| 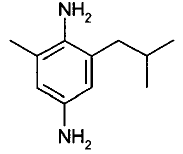 | 2-Isobutyl-6-méthyl-benzène-1,4-diamine |
| 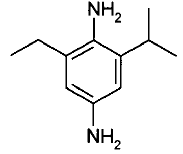 | 2-Ethyl-6-isopropyl-benzène-1,4-diamine |
| 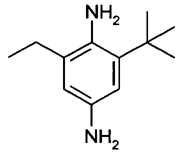 | 2-tert-Butyl-6-éthyl-benzène-1,4-diamine |
| 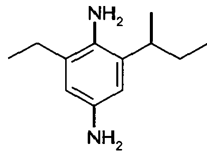 | 2-sec-Butyl-6-éthyl-benzène-1,4-diamine |
| 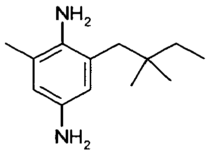 | 2-(2,2-Diméthyl-butyl)-6-méthyl-ben zene-1,4-diamine |
| 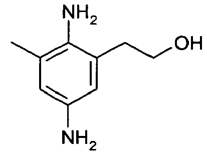 | 2-(2,5-Diamino-3-méthyl-phényl)-éthanol |
| 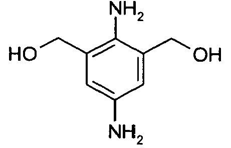 | (2,5-Diamino-3-hydroxyméthyl-phényl)-méthanol |
| 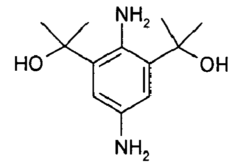 | 2-[2,5-Diamino-3-(1-hydroxy-1-méthyl-éthyl)-phényl]-propan-2-ol |
| 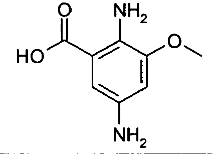 | Acide 2,5-diamino-3-méthoxy-benzoïque |
| 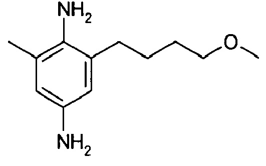 | 2-(4-Méthoxy-butyl)-6-méthyl-benzène-1,4-diamine |

4. Composé de formule (I) selon l'une quelconque des revendications précédentes, **caractérisé par le fait qu'**il est choisi parmi la 2-isopropyl-6-méthyl-benzène-1,4-diamine, la 2-éthyl-6-méthyl-benzène-1,4-diamine, la 2-tert-butyl-6-méthyl-benzène-1,4-diamine, l'acide 2,5-diamino-3-méthyl-benzoïque, 1a 2-méthoxy-6-méthyl-benzène-1,4-diamine, la 2-méthoxy-6-(2-pyrrolidin-1-yl-éthyl)-benzène-1,4-diamine, la 2-(3-diméthylamino-propyl)-6-méthyl-benzène-1,4-diamine, la 2-(3-imidazol-1-yl-propyl)-6-méthyl-benzène-1,4-diamine, le 2,5-diamino-isophthalamide, le 2,5-diamino-3-méthylbenzamide, le 1-(2,5-diamino-3-méthyl-phényl)-éthanol, la 2-méthyl-6-propyl-benzène-1,4-diamine, la 2-isobutyl-6-méthyl-benzène-1,4-diamine, le 2-(2,5-Diamino-3-méthyl-phényl)-éthanol ou l'un de leurs sels ou solvates physiologiquement acceptables.

5. Procédé de synthèse d'un composé para-phénylènediamine de formule (I) tel que défini à l'une quelconque des revendications 1 à 4, **caractérisé en ce qu'**il comprend une étape de réduction du composé intermédiaire de formule (II) correspondant ou du composé intermédiaire de formule (III) correspondant ou du composé intermédiaire de formule (IV) correspondant : 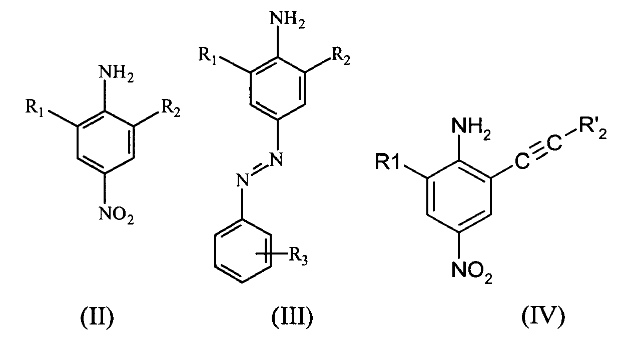 dans laquelle R₁ et R₂ représentent, indépendamment,
- un radical alkyle linéaire ou ramifié en C₁-C₆ éventuellement substitué par un radical alcoxy en C₁-C₆, hydroxy, amino, alkyle(C₁-C₆)amino, dialkyl(C₁-C₆)amino ; un radical cyclique de type :1-pyrrolidinyle, 1-cyclohexylaminyle, 1-pipérazinyle, 1-diazépanyle, 1-morpholinyle, 1-imidazoyle, ce cycle pouvant être substitué par un ou plusieurs groupements alkyle, hydroxy ou amino ;
- un radical alcoxy, linéaire ou ramifié, en C₁-C₆, monohydroxyalcoxy en C₁-C₆, polyhydroxyalcoxy en C₁-C₆;
- un radical carboxylique
- un radical amide ;
R'₂ représente indépendamment :
- un radical alkyle linéaire ou ramifié en C₁-C₄ éventuellement substitué par un radical alcoxy en C₁-C₆ , hydroxy, amino, alkyle(C₁-C₆)amino, dialkyl(C₁-C₆ )amino ou encore par un radical cyclique de type :1-pyrrolidinyle, 1-cyclohexylaminyle, 1-pipérazinyle, 1-diazépanyle, 1-morpholinyle, 1-imidazoyle, ce cycle pouvant être substitué par un ou plusieurs groupements alkyle, hydroxy ou amino ;
- un radical alcoxy, linéaire ou ramifié, en C₁-C₆ , monohydroxyalcoxy en C₁-C₆ , polyhydroxyalcoxy en C₁-C₆
R₃ étant un hydrogène, un groupe sulfonique.

6. Composé intermédiaire de formule (II) : 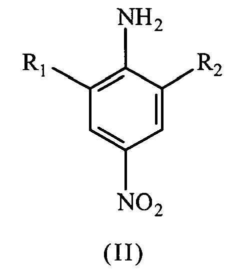 dans laquelle R₁ et R₂ représentent, indépendamment,
- un radical alkyle linéaire ou ramifié en C₁-C₆ éventuellement substitué par un radical alcoxy en C₁-C₆, hydroxy, amino, alkyle(C₁-C₆)amino, dialkyl(C₁-C₆)amino ; un radical cyclique de type :1-pyrrolidinyle, 1-cyclohexylaminyle, 1-pipérazinyle, 1-diazépanyle, 1-morpholinyle, 1-imidazoyle, ce cycle pouvant être substitué par un ou plusieurs groupements alkyle, hydroxy ou amino ;
- un radical alcoxy, linéaire ou ramifié, en C₁-C₆, monohydroxyalcoxy en C₁-C₆, polyhydroxyalcoxy en C₁-C₆;
- un radical carboxylique
- un radical amide ;
à l'exception des composés suivants

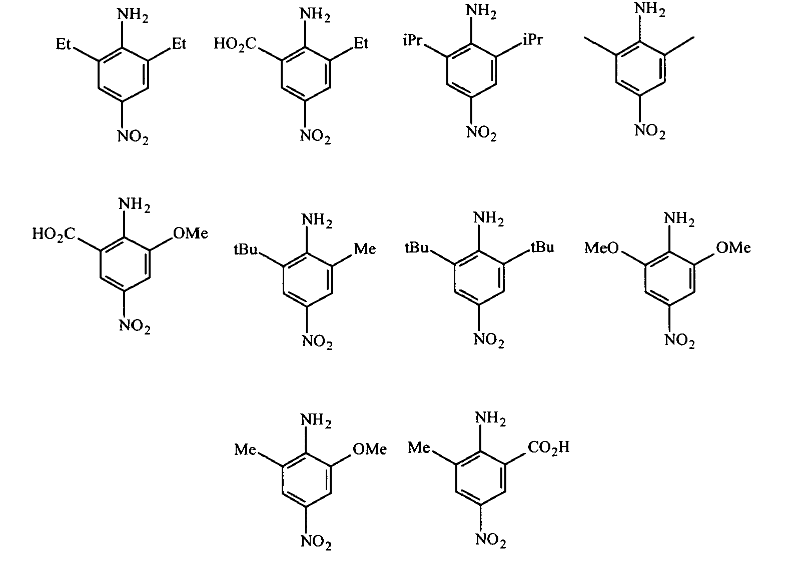

7. Composé intermédiaire de formule (III) : 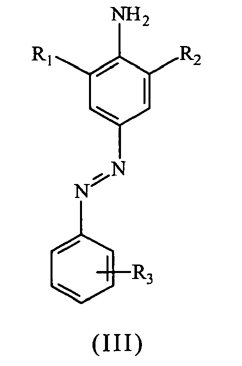 dans laquelle R₁ et R₂ représentent, indépendamment,
- un radical alkyle linéaire ou ramifié en C₁-C₆ éventuellement substitué par un radical alcoxy en C₁-C₆, hydroxy, amino, alkyle(C₁-C₆)amino, dialkyl(C₁-C₆)amino ; un radical cyclique de type :1-pyrrolidinyle, 1-cyclohexylaminyle, 1-pipérazinyle, 1-diazépanyle, 1-morpholinyle, 1-imidazoyle, ce cycle pouvant être substitué par un ou plusieurs groupements alkyle, hydroxy ou amino ;
- un radical alcoxy, linéaire ou ramifié, en C₁-C₆, monohydroxyalcoxy en C₁-C₆, polyhydroxyalcoxy en C₁-C₆;
- un radical carboxylique
- un radical amide ;
R₃ étant un hydrogène, un groupe sulfonique ;
à l'exception des composés suivants

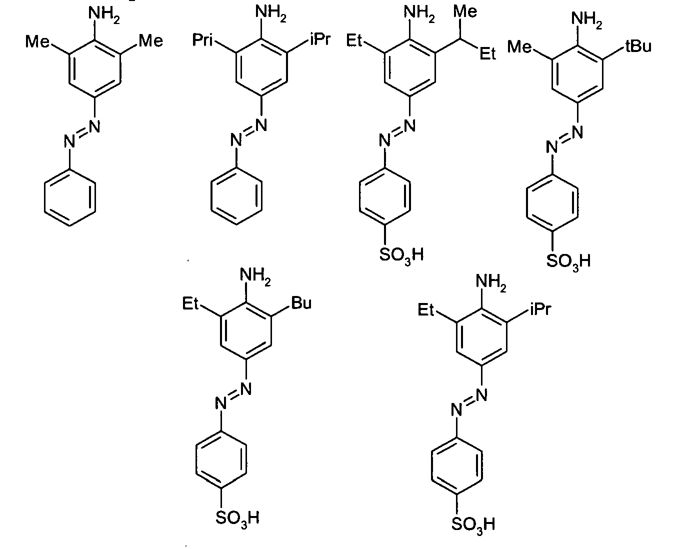

8. Composé intermédiaire de formule (IV) 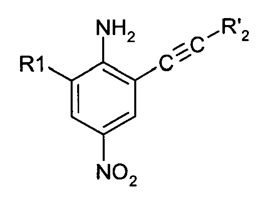 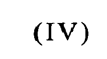 dans laquelle R₁ représente
- un radical alkyle linéaire ou ramifié en C₁-C₆ éventuellement substitué par un radical alcoxy en C₁-C₆, hydroxy, amino, alkyle(C₁-C₆)amino, dialkyl(C₁-C₆)amino ou encore par un radical cyclique de type :1-pyrrolidinyle, 1-cyclohexylaminyle, 1-pipérazinyle, 1-diazépanyle, 1-morpholinyle, 1-imidazoyle, ce cycle pouvant être substitué par un ou plusieurs groupements alkyle, hydroxy ou amino ;
- un radical alcoxy, linéaire ou ramifié, en C₁-C₆, monohydroxyalcoxy en C₁-C₆, polyhydroxyalcoxy en C₁-C₆;
- un radical carboxylique
- un radical amide ;
et R'₂ représente indépendamment :
- un radical alkyle linéaire ou ramifié en C₁-C₄ éventuellement substitué par un radical alcoxy en C₁-C₆, hydroxy, amino, alkyle(C₁-C₆)amino, dialkyl(C₁-C₆)amino ou encore par un radical cyclique de type :1-pyrrolidinyle, 1-cyclohexylaminyle, 1-pipérazinyle, 1-diazépanyle, 1-morpholinyle, 1-imidazoyle, ce cycle pouvant être substitué par un ou plusieurs groupements alkyle, hydroxy ou amino ;
- un radical alcoxy, linéaire ou ramifié, en C₁-C₆, monohydroxyalcoxy en C₁-C₆, polyhydroxyalcoxy en C₁-C₆ ;
à l'exception du composé suivant

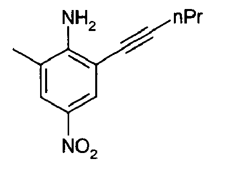

9. Composition pour la teinture d'oxydation des fibres kératiniques, **caractérisée par le fait qu'**elle contient, dans un milieu approprié pour la teinture, au moins un composé para-phénylènediamine de formule (I), ses solvates et sels d'acide physiologiquement acceptables: 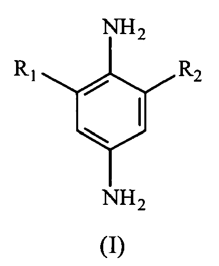 dans laquelle R₁ et R₂ représentent, indépendamment,
- un radical alkyle linéaire ou ramifié en C₁-C₆ éventuellement substitué par un radical alcoxy en C₁-C₆, hydroxy, amino, alkyle(C₁-C₆)amino, dialkyl(C₁-C₆)amino ou encore par un radical cyclique de type :1-pyrrolidinyle, 1-cyclohexylaminyle, 1-pipérazinyle, 1-diazépanyle, 1-morpholinyle, 1-imidazoyle, ce cycle pouvant être substitué par un ou plusieurs groupements alkyle, hydroxy ou amino ;
- un radical alcoxy, linéaire ou ramifié, en C₁-C₆, monohydroxyalcoxy en C₁-C₆, polyhydroxyalcoxy en C₁-C₆;
- un radical carboxylique
- un radical amide.
à l'exception des composés suivants :

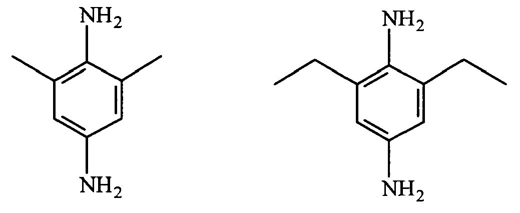

10. Composition selon la revendication précédente dans laquelle les groupes R₁ et R₂ du composé para-phénylènediamine de formule (I) représentent indépendamment
- un radical méthyle, éthyle, n-propyle, iso-propyle, sec-butyle, iso-butyle, ter-butyle, (2,2'diméthyl)butyle ;
- un radical méthoxybutyle, aminométhyle, (3-diméthylamino)propyle, imidazolo-n-propyle, pyrrolidinoéthyle ; imidazolopropyle ; diméthylaminopropyle ;
- un radical méthoxy, hydroxyméthyle, α-hydroxyéthyle, β-hydroxyéthyle, β-hydroxy-iso-propyle ;
- un radical carboxylique ;
- un radical amido.

11. Composition selon la revendication 9 ou 10, **caractérisée par le fait que** le composé para-phénylènediamine de formule (I) est choisi parmi les composés suivants : la 2-isopropyl-6-méthyl-benzène-1,4-diamine, la 2-éthyl-6-méthyl-benzène-1,4-diamine, la 2,6-diisopropyl-benzène-1,4-diamine, la 2-tert-butyl-6-méthyl-benzène-1,4-diamine, l'acide 2,5-diamino-3-méthyl-benzoïque, la 2-méthoxy-6-méthyl-benzène-1,4-diamine, la 2-méthoxy-6-(2-pyrrolidin-1-yl-ethyl)-benzène-1,4-diamine, la 2-(3-diméthylamino-propyl)-6-méthyl-benzène-1,4-diamine, la 2-(3-diméthylamino-propyl)-6-méthoxybenzène-1,4-diamine, la 2,6-bis-(3-diméthylamino-propyl)-benzène-1,4-diamine, la 2-(3-imidazol-1-yl-propyl)-6-méthyl-benzène-1,4-diamine, là 2,6-bis-aminométhyl-benzène-1,4-diamine, le 2,5-diamino-isophthalamide, (2,5-diamino-3-méthyl-phényl)-méthanol, le 2,5-diamino-3-méthylbenzamide, le 1-(2,5-diamino-3-méthyl-phényl)-éthanol, la 2-méthyl-6-propyl-benzène-1,4-diamine, la 2-isobutyl-6-méthyl-benzène-1,4-diamine, la 2-éthyl-6-isopropyl-benzène-1,4-diamine, la 2-tert-butyl-6-éthyl-benzène-1,4-diamine, la 2-sec-butyl-6-éthyl-benzène-1,4-diamine, la 2-(2,2-diméthyl-butyl)-6-méthyl-benzène-1,4-diamine, le 2-(2,5-diamino-3-méthyl-phényl)-éthanol, le (2,5-diamino-3-hydroxyméthyl-phényl)-méthanol, le 2-[2,5-diamino-3-(1-hydroxy-1-méthyl-éthyl)-phényl]-propan-2-ol, l'acide 2,5-diamino-3-méthoxy-benzoïque, la 2-(4-méthoxy-butyl)-6-méthyl-benzène-1,4-diamine ou l'un de leurs sels ou solvates physiologiquement acceptables.

12. Composition selon la revendication 11, dans laquelle le composé para-phénylènediamine de formule (I) est choisi parmi la 2-isopropyl-6-méthyl-benzène-1,4-diamine, la 2-éthyl-6-méthyl-benzène-1,4-diamine, la 2,6-diisopropyl-benzène-1,4-diamine, la 2-tert-butyl-6-méthyl-benzène-1,4-diamine, l'acide 2,5-diamino-3-méthyl-benzoïque, la 2-méthoxy-6-méthyl-benzène-1,4-diamine, la 2-méthoxy-6-(2-pyrrolidin-1-yl-éthyl)-benzène-1,4-diamine, la 2-(3-diméthylamino-propyl)-6-méthyl-benzène-1,4-diamine, la 2-(3-imidazol-1-yl-propyl)-6-méthyl-benzène-1,4-diamine, le 2,5-diamino-isophthalamide, le 2,5-diamino-3-méthylbenzamide, le 1-(2,5-diamino-3-méthyl-phényl)-éthanol, la 2-méthyl-6-propyl-benzène-1,4-diamine, la 2-isobutyl-6-méthyl-benzène-1,4-diamine, le 2-(2,5-Diamino-3-méthyl-phényl)-éthanol ou l'un de leurs sels ou solvates physiologiquement acceptables.

13. Composition selon l'une quelconque des revendications 9 à 12, **caractérisée par le fait qu'**elle contient de 0,001 à 10% en poids d'au moins un composé para-phénylènediamine de formule (I), ses sels ou ses solvates.

14. Composition selon la revendication 13, **caractérisée par le fait qu'**elle contient de 0,05 à 6% en poids, et de préférence 0,1 à 3% en poids, d'au moins un composé para-phénylènediamine de formule (I), ses sels ou ses solvates.

15. Composition selon l'une quelconque des revendications 9 à 14, **caractérisée par le fait que** le milieu approprié pour la teinture est constitué par de l'eau ou par un mélange d'eau et d'au moins un solvant organique choisi parmi les alcanols inférieurs en C₁-C₄, les polyols et éthers de polyols, les alcools aromatiques, et leurs mélanges.

16. Composition selon l'une quelconque des revendications 9 à 15, **caractérisée par le fait qu'**elle présente un pH compris entre 3 et 13.

17. Composition selon l'une quelconque des revendications 9 à 16 **caractérisée par le fait qu'**elle contient au moins une base d'oxydation additionnelle choisie parmi les para-phénylènediamines différentes des para-phénylènediamines de formule (I), les bis-phénylalkylènediamines, les para-aminophénols, les ortho-aminophénols, des bases hétérocycliques et leurs sels d'addition avec un acide.

18. Composition selon la revendication 17, **caractérisée par le fait que** la ou les bases d'oxydation additionnelles représentent de 0,0005 à 12% en poids du poids total de la composition tinctoriale.

19. Composition selon l'une quelconque des revendications 9 à 18, **caractérisée par le fait qu'**elle contient au moins un coupleur et/ou au moins un colorant direct.

20. Composition selon la revendication 19, **caractérisée par le fait que** le ou les coupleurs sont choisis parmi les méta-phénylènediamines, les méta-aminophénols, les méta-diphénols, les dérivés mono- ou polyhydroxylés du naphtalène et les coupleurs hétérocycliques et leurs sels d'addition avec un acide.

21. Composition selon l'une quelconque des revendications 19 ou 20, **caractérisée par le fait que** le ou les coupleurs représentent de 0,0001 à 10% en poids du poids total de la composition tinctoriale.

22. Composition selon l'une quelconque des revendications 9 à 21, **caractérisée par le fait que** les sels d'addition avec un acide sont choisis parmi les sels des acides suivants : chlorhydrique, bromhydrique, sulfurique, acétique, lactique, tartrique, citrique, méthanesulfonique, para-toluènesulfonique, benzènesulfonique, phosphorique ou succinique.

23. Procédé de teinture des fibres kératiniques et en particulier des fibres kératiniques humaines telles que 1es cheveux, **caractérisé par le fait que** l'on applique sur ces fibres au moins une composition tinctoriale telle que définie à l'une quelconque des revendications 9 à 22 pendant un temps suffisant pour développer la coloration désirée, soit à l'air, soit à l'aide d'un agent oxydant, éventuellement en présence de catalyseurs d'oxydation.

24. Procédé selon la revendication 23, **caractérisé par le fait que** la coloration est révélée au seul contact de l'oxygène de l'air.

25. Procédé selon la revendication 24, **caractérisé par le fait que** l'on révèle la couleur à pH acide, neutre ou alcalin à l'aide d'un agent oxydant qui est ajouté juste au moment de l'emploi à la composition tinctoriale ou qui est présent dans une composition oxydante appliquée simultanément ou séquentiellement de façon séparée.

26. Procédé selon la revendication 25, **caractérisé par le fait que** l'agent oxydant est choisi parmi le peroxyde d'hydrogène, le peroxyde d'urée, les bromates de métaux alcalins, les persels tels que les perborates et persulfates.

27. Dispositif à plusieurs compartiments, ou "kit" de teinture à plusieurs compartiments, dont un premier compartiment renferme une composition tinctoriale telle que définie à l'une quelconque des revendications 9 à 22 et un deuxième compartiment renferme une composition oxydante.

28. Utilisation du composé tel que défini à l'une quelconque des revendications 1 à 4 en tant que précurseur de colorant d'oxydation.
